# EUROPEAN PATENT APPLICATION

(11) **EP 2 420 500 A1**
(43) Date of publication of application: **22.02.2012**
(21) Application number: 10761444.8
(22) Date of filing: 08.04.2010
(51) Int. Cl.: C07D 401/12, A61K 31/4439, A61P 1/04, A61P 31/04, A61P 35/00

(54) **PYRIDINE THIO DERIVATIVE, AND PHARMACEUTICAL COMPOSITION WHICH CONTAINS SAME AND HAS ANTI-HELICOBACTER PYLORI ACTION**

(30) Priority: 09.04.2009 JP 2009094742
(71) Applicant: aRigen Pharmaceuticals, Inc., Tokyo 107-0062 (JP)
(72) Inventor: YAMAMOTO, Masaichi, Tokyo 107-0062 (JP); ITO, Masaharu, Tokyo 107-0062 (JP); TSUJII, Masahiko, Tokyo 107-0062 (JP); HAMAZAKI, Mitsunori, Tokyo 107-0062 (JP)
(74) Representative: McCluskie, Gail Wilson
(86) International application number: PCT/JP2010/002561
(87) International publication number: WO 2010/116740

(57) **Abstract**

Disclosed are a novel pyridine thio derivative, and a pharmaceutical composition using the novel pyridine thio derivative, particularly a pharmaceutical composition having selective antibacterial activity against Helicobacter pylori. Specifically disclosed is a pyridine thio derivative represented by general formula (I) (wherein R₁ and R₂ each represents a hydrogen atom, a C₁₋₈ alkyl group or the like; R₃ and R₄ each represents a hydrogen atom, a C₁₋₈ alkyl group or the like; X represents -O-, -S- or the like; Y represents a C₁₋₁₂ alkyl group which may be substituted by a substituent or -(R^{S}-O)ₙ-R⁶(wherein R⁵ represents a C₁₋₅ alkylene group; R⁶ represents a C₁₋₈ alkyl group which may be substituted by a substituent, and n represents an integer of 1-10); and Z represents a hydrogen atom or the like), or a pharmacologically acceptable salt thereof. Also specifically disclosed is a pharmaceutical composition containing the pyridine thio derivative or a salt thereof.

## Description

### TECHNICAL FIELD

The present invention relates to a novel pyridine thio derivative represented by the general formula (I) which will be described later or a pharmacologically acceptable salt thereof and to a pharmaceutical composition containing the pyridine thio derivative or a salt thereof.

### BACKGROUND ART

Gastritis, gastric ulcers and duodenal ulcers are diseases developed by complicated involvements of factors such as stresses, genetic factors and living habitudes. In recent years, attention is focused on Helicobacter pylori (H. pylori) as one of these factors. Enthusiastic studies have been made as to the relations between Helicobacter pylori and gastritis, gastric ulcers, duodenal ulcers, and gastric cancers since Warren and Marshall succeeded in the isolation culture of spiral-shaped bacteria from a biopsy of a gastric sample in 1983. As a result, there is a report concerning Helicobacter pylori infection rate, in which the Helicobacter pylori positive rate is about 4% for healthy stomach whereas the Helicobacter pylori positive rates are as high as about 83% for chronic gastritis, about 69% for gastric ulcer, about 92% for duodenal ulcer, and about 51% for non-ulcer dyspepsia syndromes (see Non-Patent Document 1). Further, the Helicobacter pylori infection also has a strong relation to the incidence of gastric cancers, and International Agency for Research on Cancer of the World Health Organization (WHO) concluded in 1994 that Helicobacter pylori was a carcinogen having a causal role in the development of gastric cancers.
Conventional medical treatments for gastritis, gastric ulcer, duodenal ulcer, and the like are mainly performed by symptomatic treatments using drugs such as a H₂ blocker and proton pump inhibitor (PPI), which suppress gastric acid secretion, mucosal protective agents, or the like. However, even if the legions of patients are temporarily cured by these drugs, it is said that about 80% of these patients will have a relapse within one year if the treatment is stopped (see Non-Patent Document 1). It is also revealed that if Helicobacter pylori (H. pylori) is eradicated, the one-year recurrence rate of duodenal ulcer is within 10% and that of gastric ulcer is clearly low (see Non-Patent Document 2) . This brings about a method in which, in addition to a proton pump inhibitor (PPI), a large dose of antibacterial agents such as amoxicillin, clarithromycin, and metronidazole are administered at the same time, over one week or more. However, the administration of a large amount of antibacterial agents results in the eradication of effective bacteria. As a result, there is a fear as to the possibility of promoting the occurrence of side effects such as soft stools, diarrhea, dysgeusia, glossitis, stomatitis, impairment of liver function, abnormal hepatic function, and hemorrhagic enteritis, and further the occurrence of methicillin-resistant Staphylococcus aureus (MRSA) .

Efforts have been exerted to develop pharmaceuticals which exhibit satisfactory antibacterial activity against Helicobacter pylori and have a high safety in a regular dose with such a background (see Patent Documents 1 to 5).
In order to produce the same effect as antibiotics on the eradication of Helicobacter pylori in clinical practice, these pharmaceuticals need to exhibit antibacterial activity equal to or higher than the anti-Helicobacter pylori activity of, for example, antibiotics exhibiting antibacterial activity against Helicobacter pylori in clinical practice. In other words, these pharmaceuticals are desired to have an activity with a concentration higher than a minimum inhibitory concentration (MIC) of 0.3 µg/ml.
Further, some compounds among guanidinomethylcyclohexane carboxylate derivatives described in Patent Document 6 each have a MIC of less than 1 µg/ml as the anti-Helicobacter pylori activity. However, each of these compounds is intrinsically decomposed very rapidly by a decomposition enzyme in the small intestine or blood. This nature is regarded to be that of compounds designed based on such metabolic characteristics that the compounds are decomposed in the small intestine or blood as an evidence of the selectivity in antibacterial activity against Helicobacter pylori from the thought described in Patent Document 7 reading as follows: "When antibiotics or synthetic antimicrobial agents are administered, they are metabolically distributed, including, for example, those which pass the digestive tract and are absorbed from the enteric canal to enter the blood and those which are excreted together with feces, with the result that a large number of bacteria living in the intestine are eradicated by the pharmaceutical passing through the enteric canal, leading to the disturbance of the balance in the gut flora and it is therefore necessary to avoid the administration over a long time.". However, metabolic enzymes in the intestine and blood and enterobacteria are known to be varied by an individual difference and foods to be taken, and it is therefore less possible that patients having a variety of backgrounds ensure such metabolic characteristics stably.
In the meantime, there are pyridine derivatives (see Patent Document 8) known as antiulcer agents, pyridine derivatives (see Patent Document 2) exhibiting antibacterial activity against Helicobacter pylori, and pyridine derivatives (see Patent Document 9) used for gastric acid secretion inhibitors. As the pyridine derivatives,
2-[{4-(2-hydroxyethoxy)-3-methylpyridine-2-yl}methylthio]-1 H-benzimidazole,
2-[{4-(3-hydroxypropoxy)-3-methylpyridine-2-yl}methylthio]-1H-benzimidazole, and the like are compounds useful for antiulcer agents in Examples 26 and 34 described in Patent Document 5. However, there is no description concerning experimental data relating to the antiulcer activity of each of these compounds and further, there is neither description nor hint concerning the activities of these compounds against Helicobacter pylori.
Further, the inventors of the present invention have found a pyridylmethylthio-1H-benzimidazole derivative having extremely strong anti-Helicobacter pylori activity (see Patent Document 10) .
Under this situation, studies have been made as to antiulcer agents, gastric acid secretion inhibitors, or pyridine derivatives having anti-Helicobacter pylori activity. Any useful compound has not been developed yet in spite of these studies and efforts.

In the meantime, these 1H-benzimidazole-2-thio derivatives can be produced easily by using, as starting material, 2-mercaptomethyl-1H-banzimidazoles which can be produced easily by using, for example, thiourea and the like as starting material. However, it is necessary that these 2-mercaptomethyl-1H-banzimidazole derivatives be produced by entirely different synthetic methods, and therefore, studies using these derivatives have been seldom made so far.
When, for example, a compound having a pyridine-2-yl-thiomethyl-1H-benzimidazole structure was searched by CAS online, the number of hits of the compound was as small as 10. Eight compounds among these ten compounds were compounds in which a nitrogen atom of benzimidazole is benzene-sulfunylated and a sulfur atom of the thio group is sulfinylated. One other compound is a compound in which a chlorine atom is bonded at the fourth position of pyridine ring (see Non-Patent Document 3). The rest is a compound which contains a methoxy group and a cyano group at the fourth and third positions of the pyridine ring respectively and is used as a raw material for synthesizing an indenofluorenone ring by cyclization of the cyano group (see Non-Patent Document 4).
The hit 10 compounds are described in two documents, which are shown as Non-Patent Documents 3 and 4.

### CITATION LIST

### PATENT DOCUMENT

Patent Document 1: Japanese Patent Application Laid-Open (JP-A) No. 2-209809
Patent Document 2: JP-A No. 3-173817
Patent Document 3: JP-A No. 3-48680
Patent Document 4: JP-A No. 7-69888
Patent Document 5: JP-A No. 5-247035
Patent Document 6: WO96/06825
Patent Document 7: WO97/23207
Patent Document 8: JP-A No. 61-50979
Patent Document 9: JP-A No. 58-39622
Patent Document 10: WO2008/075462

### NON-PATENT DOCUMENT

Non-Patent Document 1: Martin J. Blaser, Clin. Infectious Disease, 15: 386-393, 1992
Non-Patent Document 2: Graham D.Y., et al., Ann. Intern. Med., 116; 705-708, 1992
Non-Patent Document 3: Ismail, M. M., et al., Chem Papers (2004), 58(2), 117-125. (Chem. Abstr. 141: 366111)
Non-Patent Document 4: Kaigorodoves, E. A., et al., Chem. Heter. Compd. (1997), 33(6), 752-753, (Chem. Abstr. 128: 127976)

### SUMMARY OF THE INVENTION

### PROBLEMS TO BE SOLVED BY THE INVENTION

It is an object of the present invention to provide a new kind of compound having strong and selective anti-Helicobacter pylori activity. In other words, the present invention provides a novel pyridine derivative and a pharmaceutical composition using the pyridine derivative, and particularly, a pharmaceutical composition having selective antibacterial activity against Helicobacter pylori.

### MEANS FOR SOLVING THE PROBLEMS

The inventors of the present invention have focused their attentions on the crosslinked part between a pyridine ring and a 1H-benzimidazole ring and made studies concerning the correlation between the crosslinked structure and anti-Helicobacter pylori activity, and as a result, surprisingly found that a pyridine compound having a structure in which a methylene group and a thio group are replaced to each other has very strong and selective anti-Helicobacter pylori activity.
That is, the present invention relates to a pyridine thio derivative represented by the following general formula (I) or a pharmacologically acceptable salt thereof:

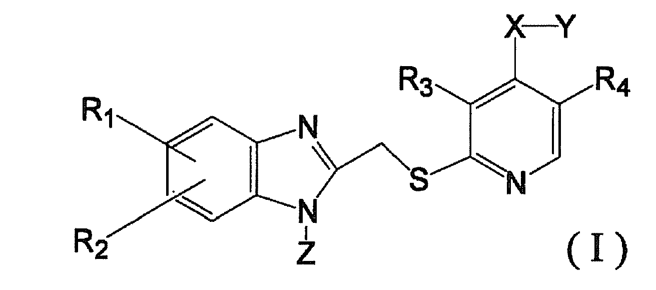

wherein R₁ and R₂ respectively represent a hydrogen atom, a C₁₋₈ alkyl group, a C₁₋₈ alkoxy group, or a halogen atom; R₃ and R₄ respectively represent a hydrogen atom, a C₁₋₈ alkyl group, or a C₁₋₈ alkoxy group; X represents -O- , -S-, or -NH- ; Y represents a C₁₋₁₂ alkyl group which may be substituted with a substituent selected from the group consisting of a hydroxyl group, a C₁₋₈ alkoxy group, and a halogen atom, or -(R⁵-O)ₙ-R⁶ (where R⁵ represents a C₁₋₅ alkylene group, R⁶ represents a C₁₋₈ alkyl group which may be substituted with a substituent selected from the group consisting of a hydroxyl group, a C₁₋₈ alkoxy group, and a halogen atom, and n denotes an integer from 1 to 10) ; and Z represents a hydrogen atom or a C₁₋₈ alkyl group.
Further, the present invention relates to a pharmaceutical composition comprising a pyridine thio derivative represented by the above general formula (I) or a pharmacologically acceptable salt thereof, and a pharmaceutically acceptable carrier, and particularly, to a pharmaceutical composition that prevents or treats Helicobacter pylori (H. pylori) infections or diseases in which Helicobacter pylori (H. pylori) is involved.
The present invention also relates to a pyridine thio derivative represented by the above general formula (I) or a pharmacologically acceptable salt thereof, as an effective component that prevents or treats Helicobacter pylori (H. pylori) infections or diseases in which Helicobacter pylori (H. pylori) is involved.
Further, the present invention relates to a method for preventing or treating diseases in which helicobacter pylori (H. pylori) is involved, the method including administering a pharmaceutical composition containing a pyridine thio derivative represented by the above general formula (I) or a pharmacologically acceptable salt thereof in an effective amount to a patient infected with Helicobacter pylori (H. pylori).
Moreover, the present invention relates to a use of a pyridine thio derivative represented by the above general formula (I) or a pharmacologically acceptable salt thereof for producing a pharmaceutical composition that prevents or treats Helicobacter pylori infections or diseases in which Helicobacter pylori is involved.

The following descriptions are to explain the present invention in more detail.
(1) A pyridine thio derivative represented by the following general formula (I) or a pharmacologically acceptable salt thereof:

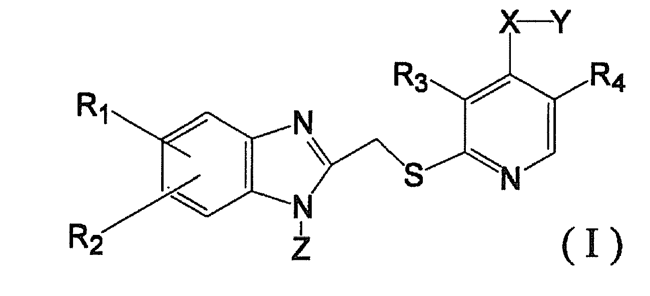

wherein R₁ and R₂ respectively represent a hydrogen atom, a C₁₋₈ alkyl group, a C₁₋₈ alkoxy group, or a halogen atom; R₃ and R₄ respectively represent a hydrogen atom, a C₁₋₈ alkyl group, or a C₁₋₈ alkoxy group; X represents -O-, -S-, or -NH-; Y represents a C₁₋₁₂ alkyl group which may be substituted with a substituent selected from the group consisting of a hydroxyl group, a C₁₋₈ alkoxy group, and a halogen atom, or -(R⁵-O)ₙ-R⁶ (where R⁵ represents a C₁₋₅ alkylene group, R⁶ represents a C₁₋₈ alkyl group which may be substituted with a substituent selected from the group consisting of a hydroxyl group, a C₁₋₈ alkoxy group, and a halogen atom, and n denotes an integer from 1 to 10); and Z represents a hydrogen atom or a C₁₋₈ alkyl group.
(2) The pyridine thio derivative or a pharmacologically acceptable salt thereof according to the above (1), wherein Y in the general formula (I) is a C₃₋₁₀ alkyl group which may be substituted with a substituent selected from the group consisting of a hydroxyl group or a C₁₋₈ alkoxy group.
(3) The pyridine thio derivative or a pharmacologically acceptable salt thereof according to the above (1), wherein Y in the general formula (I) represents -(R⁵-O)ₙ-R⁶ (where R⁵ represents a C₁₋₅ alkylene group, R⁶ represents a C₁₋₈ alkyl group which may be substituted with a substituent selected from the group consisting of a hydroxyl group, a C₁₋₈ alkoxy group, and a halogen atom, and n denotes an integer from 1 to 10), in which R⁵ is an ethylene group and R⁶ is a C₁₋₈ alkyl group which may be substituted with a fluorine atom.
(4) The pyridine thio derivative or a pharmacologically acceptable salt thereof according to any one of the above (1) to (3), wherein X in the general formula (I) is -O- or -S-.
(5) The pyridine thio derivative or a pharmacologically acceptable salt thereof according to any one of the above (1) to (4), wherein R₃ and R₄ in the general formula (I) respectively represent a hydrogen atom, a methyl group, or a methoxy group.
(6) A pharmaceutical composition, containing the pyridine thio derivative or a pharmacologically acceptable salt thereof according to any one of the above (1) to (5), and a pharmaceutically acceptable carrier.
(7) The pharmaceutical composition according to the above (6), the composition being used to prevent or treat Helicobacter pylori (H. pylori) infections.
(8) The pharmaceutical composition according to the above (6), the composition being used to prevent or treat diseases in which Helicobacter pylori (H. pylori) is involved.
(9) The pharmaceutical composition according to the above (8), wherein the diseases in which Helicobacter pylori (H. pylori) is involved are gastritis, gastric ulcer, duodenal ulcer, non-ulcer dyspepsia syndromes, gastric MALT lymphoma, hyperplastic gastric polyps, gastric cancers, gastrointestinal cancers, pancreatitis, or inflamed intestinal diseases.
(10) A method for preventing or treating diseases in which helicobacter pylori (H. pylori) is involved, the method including administering a pharmaceutical composition containing a pyridine thio derivative or a pharmacologically acceptable salt thereof according to any one of the above (1) to (5) in an effective amount to a patient infected with Helicobacter pylori (H. pylori).
(11) The method according to the above (10), wherein the diseases in which Helicobacter pylori is involved are gastritis, gastric ulcer, duodenal ulcer, non-ulcer dyspepsia syndromes, gastric MALT lymphoma, hyperplastic gastric polyps, gastric cancers, gastrointestinal cancers, pancreatitis, or inflamed intestinal diseases.
(12) A use of the pyridine thio derivative or a pharmacologically acceptable salt thereof according to any one of the above (1) to (5) for producing a pharmaceutical composition that prevents or treats Helicobacter pylori infections or diseases in which Helicobacter pylori is involved.
(13) The use according to the above (12), wherein the diseases in which Helicobacter pylori is involved are gastritis, gastric ulcer, duodenal ulcer, non-ulcer dyspepsia syndromes, gastric MALT lymphoma, hyperplastic gastric polyps, gastric cancers, gastrointestinal cancers, pancreatitis, or inflamed intestinal diseases.
(14) The pyridine thio derivative or a pharmacologically acceptable salt thereof according to any one of the above (1) to (5), the pyridine thio derivative or derivative being used as an effective component that prevents or treat Helicobacter pylori infections or diseases in which Helicobacter pylori is involved.
(15) The pyridine thio derivative or a pharmacologically acceptable salt thereof according to the above (14), wherein the diseases in which Helicobacter pylori is involved are gastritis, gastric ulcer, duodenal ulcer, non-ulcer dyspepsia syndromes, gastric MALT lymphoma, hyperplastic gastric polyps, gastric cancers, gastrointestinal cancers, pancreatitis, or inflamed intestinal diseases.

### EFFECTS OF THE INVENTION

The novel pyridine thio derivative or a pharmacologically acceptable salt thereof according to the present invention is very useful as an antibacterial agent against Helicobacter pylori that exhibits selective antibacterial activity against Helicobacter pylori without affecting bacteria and can be administered for a long period of time. Further, the compound of the present invention has a MIC of as low as 0.003 µg/ml as the anti-Helicobacter pylori activity, showing that it has very strong antibacterial activity and is therefore useful as preventive or treating agents for various diseases in which Helicobacter pylori is involved.
Further, the antibacterial agent against Helicobacter pylori according to the present invention may be used in combination with a gastric acid secretion inhibitor such as an H₂ blocker or proton pump inhibitor (PPI) and may be used as preventive or treating agents for various diseases in which Helicobacter pylori is involved such as gastritis, gastric ulcer, duodenal ulcer, non-ulcer dyspepsia syndromes, gastric MALT lymphoma, hyperplastic gastric polyps, gastric cancers, gastrointestinal cancers, pancreatitis, or inflamed intestinal diseases.

### MODES FOR CARRYING OUT THE INVENTION

The present invention will be explained in detail.
It was first found in the present invention that a pyridine thio derivative exhibits selective and strong antibacterial activity against Helicobacter pylori when a carbon atom at the second position of the benzimidazole ring is substituted with a 4-substituted-pyridyl thio group, even if the second position of the benzimidazole ring is not occupied by a heteroatom such as an oxygen atom or sulfur atom but is occupied by a carbon atom. Therefore, a hydrogen atom bonded to some position of, for example, a pyridine ring, benzimidazole ring and the like in a 2-(4'-substituted-pyridylthio-methyl)-benzimidazole derivative according to the present invention may be substituted with various substituents according to the need. It is important for the compound of the present invention that the second position of the benzimidazole ring is substituted with a 4-substituted-pyridylthio-methyl group.

Examples of the C₁₋₈ alkyl group in the present invention include linear or branched alkyl groups having 1 to 8, preferably 1 to 5 and more preferably 1 to 3 carbon atoms, for example, a methyl group, ethyl group, n-propyl group, isopropyl group, n-butyl group, isobutyl group, sec-butyl group, tert-butyl group, n-pentyl group, and the like.
Examples of the C₁₋₁₂ alkyl group represented by Y in the general formula (I) of the present invention include linear or branched alkyl groups having 1 to 12, preferably 1 to 10 or 3 to 12, and more preferably 3 to 10 carbon atoms, for example, a methyl group, n-propyl group, n-butyl group, isobutyl group, sec-butyl group, n-pentyl group, n-hexyl group, n-heptyl group, n-octyl group, n-nonyl group, and the like. Preferable examples of the C₁₋₁₂ alkyl group in the present invention include linear alkyl groups having 1 to 12, preferably 1 to 10 and more preferably 3 to 10 carbon atoms, for example, a n-propyl group, n-butyl group, n-hexyl group, n-heptyl group, n-octyl group, n-nonyl group, and the like.
Examples of the C₁₋₈ alkoxy group in the present invention include linear or branched alkyl groups having 1 to 8, preferably 1 to 5, and more preferably 1 to 3 carbon atoms, for example, a methoxy group, ethoxy group, n-propyloxy group, isopropyloxy group, n-butyloxy group, isobutyloxy group, sec-butyloxy group, tert-butyloxy group, n-pentyloxy group, and the like.
Examples of the halogen atom in the present invention include halogen atoms such as a fluorine atom, chlorine atom, bromine atom, iodine atom, and the like.
Examples of the C₁₋₅ alkylene group in the present invention include linear or branched alkylene groups having 1 to 5, preferably 2 to 5, and more preferably 2 to 4 carbon atoms, for example, a methylene group, ethylene group, n-propylene group, and the like.
In the present invention, n represents the number of repetitions of an oxyalkylene group and examples of n include integers from 1 to 10, preferably from 2 to 10 and more preferably from 2 to 5. Though the integers of n(s) may be unnecessarily a fixed value, for example, n(s) are a mixture of 2 and 3 or a mixture of 2 to 10; preferably n(s) are a fixed value.

The alkyl group, alkoxy group and the like in the present invention may be substituted with any substituent. Preferable examples of the substituent include a hydroxyl group, the aforementioned C₁₋₈ alkoxy groups, the aforementioned halogen atoms, and the like.

Preferable examples of R₁ and R₂ in the general formula (I) according to the present invention include a hydrogen atom or C₁₋₈ alkyl groups, and a hydrogen atom is more preferable.
Preferable examples of R₃ and R₄ in the general formula (I) according to the present invention include a hydrogen atom or C₁₋₈ alkyl groups. More preferable examples R₃ and R₄ include a hydrogen atom, methyl group and methoxy group.
Preferable examples of X in the general formula (I) according to the present invention include an oxygen atom or sulfur atom.
Examples of one of Y in the general formula (I) according to the present invention include, preferably, C₁₋₁₂ alkyl groups, more preferably, linear C₁₋₁₂ alkyl groups and even more preferably, linear C₃₋₁₂ alkyl groups, which may be substituted with a hydroxyl group or C₁₋₈ alkoxy group. Preferable examples of the C₁₋₈ alkoxy group as the substituent include C₁₋₃ alkoxy groups such as a methoxy group and ethoxy group. The hydroxyl group or C₁₋₈ alkoxy group is preferably bonded to a carbon atom at the terminal of the C₁₋₁₂ alkyl group though it may be bonded to a carbon atom at any position of the C₁₋₁₂ alkyl group. Accordingly, preferable examples of Y include C₁₋₁₂ alkyl groups, ω-hydroxy-C₁₋₁₂ alkyl groups and ω-C₁₋₈ alkoxy-C₁₋₁₂ alkyl groups.
Other preferable examples of Y in the general formula (I) according to the present invention include groups represented by the formula -(R⁵-O)ₙ-R⁶ (in the formula, R⁵ represents a C₁₋₅ alkylene group, R⁶ represents a C₁₋₈ alkyl group which may be substituted with a hydroxyl group or a halogen atom and n denotes an integer from 1 to 10) . More preferable examples of Y include groups represented by the formula - (CH₂CH₂-O)n-R⁶ (in the formula, R⁶ represents a C₁₋₈ alkyl group which may be substituted with a hydroxyl group or a halogen atom and n denotes an integer from 2 to 5) . Preferable examples of the halogen atom in this case include a fluorine atom and the number of the fluorine atoms is 1 to 6 and preferably 1 to 3. Preferable examples of the substitution position of the halogen atom include the terminals of the alkyl group though the halogen atom may be bonded to a carbon atom at any position of the C₁₋₈ alkyl group. Accordingly, preferable examples of R⁶ include C₁₋₈ alkyl groups, ω-mono- or poly-fluoro-C₁₋₈ alkyl groups and ω-hydroxy-C₁₋₈ alkyl groups.
Preferable examples of Z in the general formula (I) according to the present invention include a hydrogen atom.

Specific examples of the pharmacologically acceptable salt of the pyridine thio derivative represented by the general formula (I) according to the present invention include acid addition salts of the pyridine thio derivatives and inorganic acids (for example, hydrochloric acid, hydrobromic acid, hydroiodic acid, sulfuric acid, nitric acid, phosphoric acid and the like) or organic acids (for example, formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonic acid, aspartic acid, glutamic acid and the like); and the like.
Further, the pyridine thio derivative or a pharmacologically acceptable salt thereof may form a solvate with water or an organic solvent. Preferable examples of the solvate include a hydrate and various solvates (for example, solvates with alcohols such as ethanol).

As specific examples of the compound of the present invention, compounds represented by the general formula (I) in which R₁, R₂, R₄ and Z are respectively a hydrogen atom and R₃ is a methyl group or pharmacologically acceptable salts thereof are shown in the following Table 1.

[Table 1]

**Table 1 Compound**

| Example No | X | Y | Salt |
|---|---|---|---|
| 1 | S | (CH₂)₄-OCH₃ | HCl |
| 2 | S | (CH₂)₈-OH | H |
| 3 | S | (CH₂)₈-OCH₃ | HCl |
| 4 | O | (CH₂)₂-CH₃ | HCl |
| 5 | S | CH₃ | HCl |
| 6 | S | (CH₂)₂-CH₃ | HCl |
| 7 | S | (CH₂)₄-CH₃ | HCl |
| 8 | S | (CH₂CH₂O)₂-CH₂CF₃ | HCl |
| 9 | O | (CH₂CH₂O)₂-CH₂CF₃ | HCl |
| 10 | O | (CH₂)₄-CH₃ | HCl |
| 11 | S | (CH₂)₃-OCH₂CH₃ | H |
| 12 | O | (CH₂CH₂O)₂-CH₃ | HCl |
| 13 | O | (CH₂)₈-OH | H |
| 14 | O | (CH₂)₈-OCH₃ | HCl |

The compound represented by the general formula (I) according to the present invention or a pharmacologically acceptable salt thereof may be produced according to the method described in Examples which will be described later. These compounds may be obtained by producing a 1,2-dihydropyridine-N-oxide-2-thion derivative (4) from a pyridine-N-oxide derivative (1) by a method shown below.

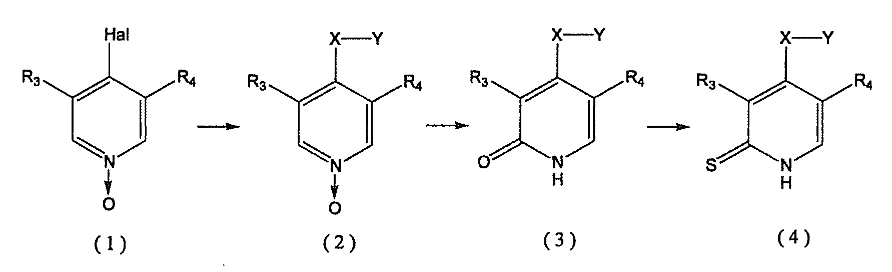

(in the formula, R₃, R₄, X and Y represent those mentioned above and Hal represents a halogen atom), and then, by reacting the 1,2-dihydropyridine-N-oxide-2-thion derivative (4) with a 2-substituted methyl-benzimidazole derivative (5) by the following method:

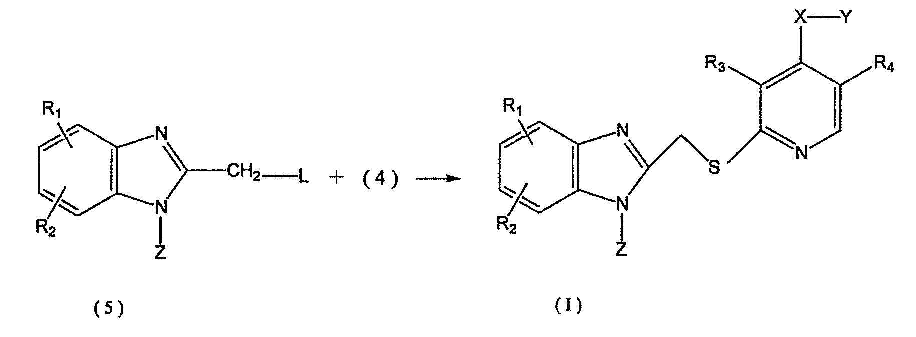

(in the formula, R₁, R₂, R₃, R₄, X, Y, and Z represent those mentioned above and L represents a leaving group.).

First, a pyridine derivative is converted into a N-oxide (1) and then, Y-XH is reacted with the N-oxide (1) to produce a pyridine derivative (2) in which -X-Y is bonded to a carbon atom at the fourth position of the pyridine ring. In this case, when X is an oxygen atom, Y-XH may be reacted in the presence of a base such as sodium hydroxide into a 4-X-Y substituted body (2) in one stage. However, when X is a sulfur atom, it is preferable that the pyridine derivative be converted into a 4-mercapto body by using, for example, a sodium bisulfide and the like, and then the 4-mercapto body is converted into a 4-S-Y substituted body by using an halogenated compound such as Y-Hal (wherein Hal represents a halogen atom) . As the halogen atom in the formula (1) and Y-Hal, a chlorine atom is preferable.

Then, the obtained 4-X-Y substituted body (2) is converted into a dihydropyridin-2-one body (3) in the presence of acetic acid anhydride and the like, which is then converted into a sulfide by using a Lawesson' s reagent to thereby produce a dihydropyridin-2-thione body (4).
The obtained dihydropyridin-2-thione body (4) may be reacted with a benzimidazole derivative (5) to produce a target compound represented by the general formula (I) . As the leaving group L in the benzimidazole derivative (5), a halogen atom is preferable and particularly, a chlorine atom is preferable.
When an active functional group exists in these reactions, it is preferable to protect the functional group by using a protective group according to the need to run reaction.

The compound represented by the general formula (I) in the present invention or a salt thereof can eradicate or make bacteriostatic Helicobacter pylori in the bodies of animals belonging to mammals such as a human and is therefore effective as an anti-Helicobacter pylori agent.
A pharmaceutical agent containing the compound represented by the general formula (I) or a salt thereof according to the present invention is effective to prevent or treat diseases in which Helicobacter pylori is involved. "The diseases in which Helicobacter pylori is involved" in the present invention mean diseases elicited or exacerbated by the infection, existence or proliferation of Helicobacter pylori in living bodies. In other words, "The diseases in which Helicobacter pylori is involved" are diseases of which the symptoms are possibly be ameliorated by eliminating Helicobacter pylori. Examples of these diseases include gastritis, gastric ulcer, duodenal ulcer, non-ulcer dyspepsia syndromes, gastric MALT lymphoma, hyperplastic gastric polyps, and gastric cancers (especially, gastric cancers which relapse after endoscopic ablation of early gastric cancers). Other examples of "The diseases in which Helicobacter pylori is involved" include gastrointestinal cancers and pancreatitis caused by Helicobacter pylori. The compound or a salt thereof according to the present invention can retard or inhibit the progress of gastrointestinal cancers associated with Helicobacter pylori. Further examples of "The diseases in which Helicobacter pylori is involved" include inflamed intestinal diseases caused by Helicobacter pylori.

Preventive or treating agents for diseases in which helicobacter pylori is involved are compositions containing, as effective components, the pyridine derivative represented by the general formula (I) or a pharmacologically acceptable salt thereof according to the present invention and may be used as pharmaceutical compositions. In this case, the compound according to the present invention, a pharmacologically acceptable carrier and/or additives are usually formulated and used as a pharmaceutical composition, though the compound according to the present invention may be used independently.
In the pharmaceutical composition of the present invention, various dosage forms may be adopted corresponding to prophylactic and therapeutic purposes, and examples of the dosage form include powders, fine granules, granules, tablets, capsules, dry syrups, syrups, injections, and the like. These dosage forms may be administered orally or parenterally.

When an oral solid formulation is prepared, an excipient and, as required, a binder, a disintegrator, a lubricant, a colorant, a corrective/corrigent, and the like may be added to the compound of the present invention, and then the obtained composition may be made into tablets, coated tablets, granules, powders, capsules, or the like by the usual method. The additives may be those usually used in the fields concerned. For example, corn starch, lactose, saccharose, sodium chloride, mannitol, sorbitol, glucose, starch, calcium carbonate, kaolin, microcrystal cellulose, silicic acid or the like may be used as the excipient. Water, ethanol, gum arabic, traganth, propanol, simple syrup, glucose solution, starch solution, gelatin solution, carboxymethyl cellulose, hydroxypropyl cellulose, gelatin, hydroxypropyl starch, methyl cellulose, ethyl cellulose, shellac, calcium phosphate, polyvinyl alcohol, polyvinyl ether, polyvinyl pyrrolidone, or the like may be used as the binder. Gelatin powders, crystal cellulose, dry starch, sodium alginate, pectin, agar powders, carboxymethyl cellulose, sodium bicarbonate, calcium carbonate, calcium citrate, sodium laurylsulfate, stearic acid monoglyceride, lactose, or the like may be used as the disintegrator. Silica, refined talc, stearate, borax, polyethylene glycol, or the like may be used as the lubricant. As the colorant, colorants such as titanium oxide and iron oxide which are permitted to be added may be used. As the corrective/corrigent, saccharose, bitter orange peel, citric acid, tartaric acid, or the like may be used.
In the case of preparing an oral liquid formulation, a corrective, a buffer, a stabilizer, a corrigent and the like may be added to the compound of the present invention to produce liquid preparation for oral administration, syrup, elixir and the like by the usual method. In this case, the corrective/corrigent may be those mentioned above. Examples of the buffer include sodium citrate and the like. Examples of the stabilizer include traganth, gum arabic, gelatin, and the like.

In the case of preparing an injection, a pH regulator, a buffer, a stabilizer, an isotonic agent, a local analgesic and the like may be added to the compound of the present invention to produce a subcutaneous, intramuscular or intravenous injection by the usual method. Examples of the pH regulator and buffer in this case include sodium citrate, sodium acetate, sodium phosphate, and the like. Examples of the stabilizer include sodium pyrosulfite, EDTA, thioglycolic acid, thiolactic acid, and the like. Examples of the local analgesic include procaine hydrochloride, lidocaine hydrochloride, and the like. Examples of the isotonic agent include sodium chloride, glucose, and the like.

The pharmaceutical composition and preventive or treating agents of the present invention may further contain one or two or more kinds of dextrins in addition to the pyridine thio derivative represented by the general formula (I) or a pharmacologically acceptable salt thereof. Examples of the above dextrin usable in the present invention include, but not limited to, a-dextrin, β-dextrin, γ-dextrin, α-cyclodextrin, β-cyclodextrin, γ-cyclodextrin, and the like.
The pharmaceutical composition and preventive or treating agents of the present invention may further contain one or two or more kinds of medical components suppressing gastric acid secretion in addition to the pyridine thio derivative represented by the general formula (I) or a pharmacologically acceptable salt thereof, or may be used in combinations with these medical components. Examples of the medical components suppressing gastric acid secretion include a H₂ blocker, proton pump inhibitor (PPI), and the like. Examples of the H₂ blocker which may be used in the present invention include famotidine, ranitidine, and the like. Further, examples of the proton pump inhibitor which may be used in the present invention include, but not limited to, lansoprazole, omeprazole, rabeprazole, pantoprazole, and the like.
According to the formulation combined as mentioned above, the effect of the present invention is expected to be more improved.
Though the amount of the compound of the present invention which is to be formulated in the above each dosage unit form depends, for example, on the symptom of a patient to which the compound is applied, on its dosage form, and the like, and is not therefore fixed, the amount per dosage unit form is desirably about 1 to 1200 mg in the case of an oral medicine and about 0.1 to 500 mg in the case of an injection. Further, though the dose of a medicine having the above-mentioned dosage form per day differs depending on the symptom, weight, age, sex and the like of a patient and is not defined unconditionally, it is usually about 0.1 to 5000 mg and preferably 1 to 1200 mg for adults per day and the medicine is preferably administered at a time or separately about two to four times per day.

The present invention will be explained in detail by way of Examples, which are not intended to be limiting of the present invention.

### Reference Example 1: Production of 4-mercapto-3-methylpyridine-1-oxide

130.6 g (0.910 mol, 1.0 equivalent) of 4-chloro-3-methylpyridine-1-oxide and 330.0 g (3.788 mol, 4.2 equivalent) of sodium hydrogensulfide·trihydrate were added to 2.17 L of ethanol and the mixture was reacted under reflux with stirring for 5.5 hr. The reaction solution was cooled and was distilled under reduced pressure to remove a solvent to thereby obtain a residue, to which 1.31 L of water was added to dissolve the residue. 379.2 g (3.64 mol, 4.0 equivalent) of 35% hydrochloric acid was gradually added to the dissolved solution with stirring to precipitate crystals. The obtained crystals were corrected by filtration, followed by drying under reduced pressure to obtain 124.4 g of
4-mercapto-3-methylpyridine-1-oxide (HPLC: 97.7 Area%, yield: 96.9%) .
¹H-NMR (400 MHz, CDCl₃) δ:
0.94 (3H, t J= 7Hz), 1.35-1.45 (4H, m), 1.76-1.81 (2H, m), 2.13 (3H, s), 3.97 (2H, t J= 6Hz), 4.71 (1H, d J = 14 Hz), 4.82 (1H, d J = 14 Hz), 6.69 (1H, d J = 6 Hz), 7.33-7.28 (2H, m), 7.63 (2H, m), 8.30 (1H, d J = 6 Hz)
MS m/z : 357 (M⁺)

### Example 1

### Production of 2-[[[4-[(4-methoxy-butyl]thio]-3-methyl-2-pyridyl]thio]meth yl]-1H-benzimidazole hydrochloride

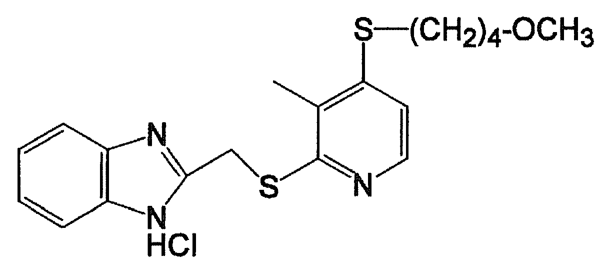

### (1) Production of 4-[(4-methoxy-butyl)thio-3-methylpyridine-1-oxide

173. 3 g (1.5 equivalent) of an aqueous 30% sodium hydroxide solution, 106.7 g (1.0 equivalent) of 1-chloro-4-methoxybutane and 1.85 L of ethanol were added to 122.8 g (0.870 mol, 1.0 equivalent) of 4-mercapto-3-methylpyridine-1-oxide produced in Reference Example 1 and the mixture was refluxed with stirring for 7 hr, and then the reaction was stopped. After the reaction mixture was cooled, it was distilled at 40°C or less under reduced pressure to remove a solvent to obtain a residue, and to the obtained residue, 0.93 L of water was added, and then the mixture was extracted with 1.5 L of ethyl acetate and then 1.0 L (x 2) of dichloromethane. The obtained organic phases were joined together and then dried by sodium sulfate anhydride. The dried organic phase was distilled at 40°C or less under reduced pressure to remove a solvent to thereby obtain 183.2 g of 4-[(4-methoxy-butyl)thio-3-methylpyridine-1-oxide.

### (2) Production of 4-[(4-methoxy-butyl)thio-2-keto-3-methylpyridine

1796.8 g (17.60 mol, 22.1 equivalent) of acetic acid anhydride was added to 181. 0 g (0.796 mol, 1. 0 equivalent) of (4-[(4-methoxy-butyl)thio-3-methylpyridine-1-oxide to react under heating/refluxing for 10 hr with stirring. The reaction solution was concentrated at 40°C or less under reduced pressure and then, 1.5 L of ethyl acetate and 0.24 L of methanol were added to the concentrated mixture, which was further stirred under heating/refluxing for 6 hr and 20 min. The reaction solution was concentrated at 40°C or less under reduced pressure and the obtained concentrated residue was refined by silica gel column chromatography to obtain 100.6 g of
4-[(4-methoxy-butyl)thio-2-keto-3-methylpyridine as an oily substance.

### (3) Production of 4-[(4-methoxy-butyl)thio]-3-methylpyridine-2-thione

96.3 g (0.238 mol, 1.1 equivalent) of a Lawesson's reagent and 0.76 L of toluene were added to 100.0 g (0.440 mol, 1.0 equivalent) of
4-[(4-methoxy-butyl)thio-2-keto-3-methylpyridine and the mixture was stirred under heating/refluxing for 14 hr. After the reaction mixture was cooled, the precipitated solid was washed with dichloromethane to thereby obtain 17.1 g of 4-[(4-methoxy-butyl)thio]-3-methylpyridine-2-thione.

### (4) Production of 2-[[[4-[(4-methoxy-butyl)thio]-3-methyl-2-pyridyl]thio]meth yl]-1H-benzimidazole hydrochloride

10.02 g (41.2 mmol, 1.0 equivalent) of
4-[(4-methoxy-butyl)thio]-3-methylpyridine-2-thione and 7.50 g (45.0 mmol, 1.1 equivalent) of 2- (chloromethyl) benzimidazole were added in a mixed solution of 6.0 g of an aqueous 30% sodium hydroxide solution and 315 mL of ethanol, and the mixture was stirred at 50°C for 6 hr. In order to complete the reaction, 1.40 g (8.4 mmol, 0.2 equivalent) of
2-(chloromethyl)benzimidazole and 1.1 g of an aqueous 30% sodium hydroxide solution were further added to the reaction solution, which was further stirred at 50°C for 4 hr, to complete the reaction. After the reaction solution was concentrated under reduced pressure, 240 mL of water was added to the reaction solution and the reaction solution was extracted with dichloromethane three times (470 mL, 200 mL, 200 mL). After the organic phase was dried by magnesium sulfate anhydride, the solvent was concentrated to dryness. The obtained residue was refined by silica gel column chromatography. A residue obtained by concentrating the target fraction after the chromatography was crystallized from ethanolic hydrochloric acid solution to obtain 9.23 g of a colorless crystal of the target
2-[[[4-[(4-methoxy-butyl)thio]-3-methyl-2-pyridyl]thio]meth yl]-1H-benzimidazole hydrochloride.
The total yield was 4.7% and the purity measured by HPLC was 99.2%.

¹H-NMR (400 MHz, DMSO-d₆) δ:
1.62-1.66 (4H, m), 2.21 (3H, s), 3.04-3.07 (2H, m), 3.21 (3H, s), 3.31-3.34 (2H, m), 4.94 (3H, s), 7.09 (1H, d J = 5.6 Hz), 7.50-7.55 (2H, m), 7.74-7.79 (2H, m), 8.14 (1H, d J = 5.6 Hz)
MS m/z : 373 (M⁺: free body)

### Example 2

### Production of 2-[[[4-[(8-hydroxy-octyl)thio]-3-methyl-2-pyridyl]thio]meth yl]-1H-benzimidazole

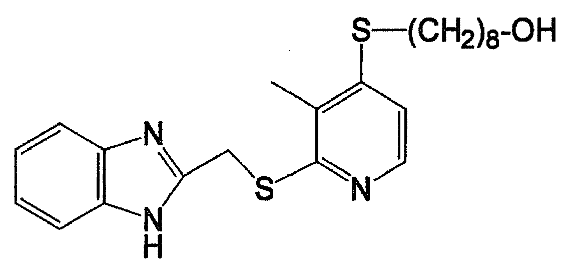

### (1) Production of 4-[(8-hydroxy-octyl)thio]-3-methylpyridine-1-oxide

88.7 g (1.6 equivalent) of an aqueous 30% sodium hydroxide solution, 88.7 g (1.0 equivalent) of 8-bromo-1-octanol and 0.9 L of ethanol were added to 59.85 g (0.424 mol, 1.0 equivalent) of 4-mercapto-3-methylpyridine-1-oxide produced in Reference Example 1 and the mixture was refluxed with stirring for 5 hr, and then the reaction was stopped. After the reaction mixture was cooled, it was distilled at 40°C or less under reduced pressure to remove a solvent to obtain a residue, to the obtained residue, 0.76 L of water and then 1.5 L of ethyl acetate were added. The precipitate was collected by filtration and dried under reduced pressure to thereby obtain 103.6 g of 4-[(8-hydroxy-octyl)thio]-3-methylpyridine-1-oxide.

### (2) Production of 4-[(8-acetoxy-octyl)thio]-2-keto-3-methylpyridine

55.0 g (15.2 equivalent) of acetic acid anhydride was added to 97.43 g (0.362 mol, 1.0 equivalent) of 4-[(8-hydroxy-octyl)thio]-3-methylpyridine-1-oxide to reflux with stirring for 7 hr. After the reaction solution was distilled to remove acetic acid anhydride, 520 mL of ethyl acetate and 69 mL of methanol were added to the reaction solution, the resultant reaction solution was further refluxed with stirring for 5 hr and 30 min. The reaction solution was concentrated under reduced pressure and the obtained concentrated residue was refined by silica gel column chromatography to obtain 46.24 g of
4-[(8-acetoxy-octyl)thio]-2-keto-3-methylpyridine as an oily substance.

### (3) Production of 4-[(8-acetoxy-octyl)thio]-3-methylpyridine-2-thione

32.5 g (0.080 mol, 1.1 equivalent) of a Lawesson's reagent and 255 mL of toluene were added to 46.13 g (0.148 mol, 1.0 equivalent) of
4-[(8-acetoxy-octyl)thio]-2-keto-3-methylpyridine and the mixture was stirred under heating/refluxing for 12 hr. After the reaction mixture was cooled, the reaction mixture was distilled at 40°C or less under reduced pressure to remove a solvent. 600 mL of water was then added to the reaction solution, which was extracted with 6000 mL of dichloromethane. The obtained organic phases were joined together, washed three times with 600 mL of water, and then dried by sodium sulfate anhydride. The dried organic phase was then distilled at 40°C or less under reduced pressure to remove a solvent and the obtained concentrated residue was refined by silica gel column chromatography to thereby obtain 8.40 g of
4-[(8-acetoxy-octyl)thio]-3-methylpyridine-2-thione.

### (4) Production of 2-[[[4-[(8-hydroxy-octyl)thio]-3-methyl-2-pyridyl]thio]meth yl]-1H-benzimidazole

8.40 g (25.6 mmol, 1.0 eq.) of
4-[(8-acetoxy-octyl)thio]-3-methylpyridine-2-thione and 4.32 g (25.9 mmol, 1.0 eq.) of 2-(chloromethyl)benzimidazole were added in a mixed solution of 3.20 g of an aqueous 30% sodium hydroxide solution and 192 mL of ethanol, and the mixture was stirred at 50°C for 2 hr and 30 min. Because the reaction was not completed, 0.76 g (4.6 mmol, 0.2 eq.) of
2-(chloromethyl)benzimidazole and 0.56 g of an aqueous 30% sodium hydroxide solution were added to the reaction solution, which was further reacted at 50°C for 1 hr. In order to complete the reaction, 1.53 g (9.2 mmol, 0.4 eq.) of 2-(chloromethyl)benzimidazole and 1.15 g of an aqueous 30% sodium hydroxide solution were added again to the reaction solution, which was further stirred at 20°C for 13 hr. 3.21 g of an aqueous 30% sodium hydroxide solution was further added to the reaction solution, which was then further stirred at 20°C for 10 hr. The precipitate was recrystallized from aqueous acetone to obtain 6.45 g of a colorless crystal of the target 2-[[[4-[(8-hydroxy-octyl)thio]-3-methyl-2-pyridyl]thio]meth yl]-1H-benzimidazole.
The total yield was 3.9% and the purity measured by HPLC was 98.4%.

¹H-NMR (400 MHz, DMSO-d₆) δ:
1.41-1.26 (10H, m), 1.63 (2H, m), 2.17 (3H, s), 3.04 (2H, t, J =7.2 Hz), 3.37-3.34 (2H, m), 4.34 (1H, bs), 4.66 (2H, s), 7.08 (1H, d J = 5.6 Hz), 7.12-7.15 (2H, m), 7.48 (2H, bs), 8.25 (1H, d J = 5.6 Hz), 12.25 (1H, bs)
MS m/z : 415 (M⁺)

### Example 3

### Production of 2-[[[4-[(8-methoxy-1-octyl]thio]-3-methyl-2-pyridyl]thio]me thyl]-1H-benzimidazole hydrochloride

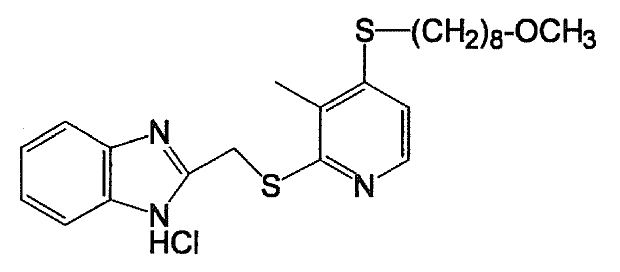

### (1) Production of 4-[(8-methoxy-octyl)thio-3-methylpyridine-1-oxide

2.25 g (1.5 equivalent) of an aqueous 30% sodium hydroxide solution, 1.93 g (1.0 equivalent) of 8-chloro-1-methoxyoctane and 30 mL of ethanol were added to 1.6 g (11.3 mmol, 1.0 equivalent) of 4-mercapto-3-methylpyridine-1-oxide produced in Reference Example 1 and the mixture was refluxed with stirring for 19 hr, and then the reaction was stopped. After the reaction mixture was cooled, it was concentrated at 40°C or less under reduced pressure to remove a solvent to obtain a residue, to the obtained residue, 20 mL of water was added and then the mixture was extracted twice with 400 mL of ethyl acetate. The obtained organic phases were joined together and then dried by sodium sulfate anhydride. The dried organic phase was then distilled at 40°C or less under reduced pressure to remove a solvent to thereby obtain 2.36 g of
4-[(8-methoxy-octyl)thio-3-methylpyridine-1-oxide as an oily substance.

### (2) Production of 4-[(8-methoxy-octyl)thio]-2-keto-3-methylpyridine

19.4 g (22.9 equivalent) of acetic acid anhydride was added to 2.36 g (8.3 mmol, 1.0 equivalent) of
4-[(8-methoxy-octyl)thio-3-methylpyridine-1-oxide to reflux for 28 hr with stirring. The reaction solution was distilled to remove acetic acid anhydride, then 18 mL of ethyl acetate and 2.5 mL of methanol were added to the reaction solution, and the reaction solution was further refluxed with stirring for 11 hr. The reaction solution was concentrated under reduced pressure and the obtained concentrated residue was refined by silica gel column chromatography to obtain 1.41 g of
4-[(8-methoxy-octyl)thio]-2-keto-3-methylpyridine as an oily substance.

### (3) Production of 4-[(8-methoxy-octyl)thio]-3-methylpyridine-2-thione

1.10 g (2.7 mmol, 1.1 equivalent) of a Lawesson' s reagent and 10 mL of toluene were added to 1.40 g (4.9 mmol, 1.0 equivalent) of
4-[(8-methoxy-octyl)thio-2-keto-3-methylpyridine, and the mixture was stirred under heating/refluxing for 4 hr. After the reaction mixture was cooled, it was distilled at 40°C or less under reduced pressure to remove a solvent. The obtained concentrated residue was refined by silica gel column chromatography to thereby obtain 0.18 g of
4-[(8-methoxy-octyl)thio]-3-methylpyridine-2-thione.

### (4) Production of 2-[[[4-[(8-methoxy-1-octyl)thio]-3-methyl-2-pyridyl]thio]-m ethyl]-1H-benzimidazole hydrochloride

111.6 mg (0.37 mmol, 1.0 equivalent) of
4-[(8-methoxy-octyl)thio]-3-methylpyridine-2-thione and 84.1 mg (0.5 mmol, 1.0 equivalent) of 2- (chloromethyl) benzimidazole were added in a mixed solution of 0.068 mL of an aqueous 30% sodium hydroxide solution and 3 mL of ethanol, and the mixture was stirred at 50°C for 5 hr. Because the reaction was not completed, 13.6 mg (0.08 mmol, 0.2 equivalent) of
2-(chloromethyl)benzimidazole and 0.011 mL of an aqueous 30% sodium hydroxide solution were further added to the reaction solution, which was further reacted at 50°C for 14 hr and 30 min. 6 mL of water was added to the reaction solution and the reaction solution was extracted with 18 mL of dichloromethane three times. After the organic phase was dried by magnesium sulfate anhydride, the solvent was concentrated to dryness. The obtained residue was refined by silica gel column chromatography. A residue obtained by concentrating the target fraction after the chromatography was crystallized from ethanolic hydrochloric acid solution to obtain 66.5 mg of a colorless crystal of the target
2-[[[4-[(8-methoxy-octyl)thio]-3-methyl-2-pyridyl]thio]-met hyl]-1H-benzimidazole hydrochloride.
The total yield was 2.3% and the purity measured by HPLC was 97.3%.

¹H-NMR (400 MHz, DMSO-d₆) δ:
1.42-1.22 (10H, m), 1.61 (2H, m J = 6.8 Hz) 2.21 (3H, s), 3.02 (2H, t J = 7.2 Hz), 3.19 (3H, s), 3.27 (2H, t J = 6.8 Hz), 4.92 (2H, s), 7.08 (1H, d J = 5.6 Hz), 7.50-7.54 (2H, m), 7.73-7.78 (2H, m), 8.12 (1H, d J = 5.2 Hz)
MS m/z : 429 (M⁺: free body)

### Example 4

### Production of 2-[[(4-n-propoxy-3-methyl-2-pyridyl)thio]-methyl]-1H-benzim idazole hydrochloride

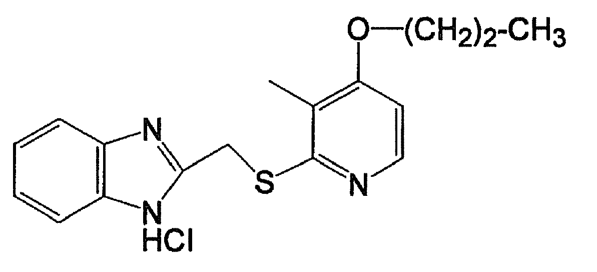

### (1) Production of 4-propoxy-3-methylpyridine-1-oxide

8.32 g (208 mmol, 2.0 equivalent) of 60% hydrogenated sodium was added to a mixed solution of 12.5 g (208 mmol, 1.0 equivalent) of 1-propanol and 200 mL of DMSO over 1 hr in a nitrogen stream while stirring the mixed solution under heating at 50°C. After the completion of the adding, and after stirring the mixed solution under heating at 53 to 57°C, 15.0 g (104.5 mmol, 1.0 equivalent) of 4-chloro-3-methylpyridine-1-oxide was added to the mixture. The mixture was further stirred under heating at 44 to 46°C for 3 hr, 15 mL of water was added and then distilled under reduced pressure to remove a solvent. After 400 mL of water was added to the concentrated residue, the solution was extracted with 500 mL of dichloromethane and then with 400 mL of dichloromethane. The extract was dried by magnesium sulfate anhydride, and then concentrated under reduced pressure to dryness to thereby obtain 24.1 g of 4-propoxy-3-methylpyridine-1-oxide.

### (2) Production of 4-propoxy-2-keto-3-methylpyridine

233.6 g (2.29 mol, 16 equivalent) of acetic acid anhydride was added to 24.0 g (143.5 mmol, 1.0 equivalent) of 4-propoxy-3-methylpyridine-1-oxide to react at 110 to 112°C for 8 hr. The reaction solution was distilled to remove acetic acid anhydride, and then 244 mL of ethyl acetate and 31 mL of methanol were added to the obtained concentrated residue, the reaction solution was further refluxed with stirring for 2 hr. The reaction solution was cooled and distilled at 40°C or less under reduced pressure to remove a solvent. The obtained concentrated residue was refined by silica gel column chromatography to obtain 9.67 g of
4-propoxy-2-keto-3-methylpyridine as an oily substance.

### (3) Production of 4-propoxy-3-methylpyridine-2-thione

11.8 g (29.2 mmol, 1.1 eq.) of a Lawesson's reagent and 93 mL of toluene were added to 9.0 g (53.8 mmol, 1. 0 equivalent) of 4-propoxy-2-keto-3-methylpyridine and the mixture was stirred under heating/refluxing for 9 hr. 1.3 g (3.2 mmol, 0.1 eq.) of a Lawesson' s reagent was further added to the reaction solution, which was reacted under refluxing for 10 hr. After the reaction solution was cooled, it was distilled at 40°C or less under reduced pressure to remove a solvent and the obtained concentrated residue was refined by silica gel column chromatography to thereby obtain 0.39 g of
4-propoxy-3-methylpyridine-2-thione.

### (4) Production of 2-[[(4-n-propoxy-3-methyl-2-pyridyl)thio]-methyl]-1H-benzim idazole hydrochloride

370 mg (2.0 mmol, 1.0 equivalent) of 4-propoxy-3-methylpyridine-2-thione and 370 mg (2.2 mmol, 1.1 equivalent) of 2-(chloromethyl)benzimidazole were added in a mixed solution of 297 mg of an aqueous 30% sodium hydroxide solution and 16 mL of ethanol, and the mixture was stirred at 50°C for 2 hr. After the reaction solution was concentrated at 40°C or less under reduced pressure, 26 mL of water was added to the solution, which was then extracted with 112 mL of dichloromethane. After the organic phase was dried by magnesium sulfate anhydride, the solvent was concentrated to dryness. The obtained residue was refined by silica gel column chromatography. A residue obtained by concentrating the target fraction after the chromatography was crystallized from ethanolic hydrochloric acid solution to obtain 431 mg of a colorless crystal of the target
2-[[(4-propoxy-3-methyl-2-pyridyl)thio]-methyl]-1H-benzimid azole hydrochloride.
The total yield was 0.14% and the purity measured by HPLC was 98.7%.

¹H-NMR (400 MHz, CDCl₃) δ:
1.09 (3H, t J = 7.2 Hz), 1.90-1.99 (2H, m), 2.40 (3H, s), 4.22 (2H, t J = 6.4 Hz), 5.46 (2H, s), 7.11 (1H, d J = 6.8 Hz), 7.48-7.53 (2H, m), 7.79-7.83 (2H, m), 8.55 (1H, d J = 6.8 Hz) MS m/z : 313 (M⁺: free body)

### Example 5

### Production of 2-[[(4-thiomethyl-3-methyl-2-pyridyl)thio]-methyl]-1H-benzi midazole hydrochloride

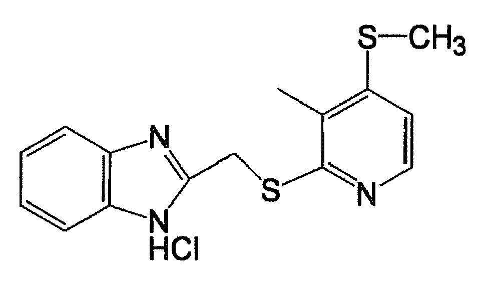

### (1) Production of 4-methylthio-3-methylpyridine-1-oxide

A mixed solution containing 5.0 g (34.8 mmol, 1.0 equivalent) of 4-chloro-3-methylpyridine-1-oxide, 2.4 g (34.2 mmol, 1.0 equivalent) of sodium thiomethoxide and 74 mL of ethanol was stirred under heating/refluxing for 5 hr. 1.2 g (17 mmol, 0.5 equivalent) of sodium thiomethoxide was further added to the solution, which was then further reacted under heating/refluxing for 1 hr and then cooled to ambient temperature. After the reaction solution was concentrated at 40°C under reduced pressure and 20 mL of water was added, the reaction solution was extracted twice with 37 mL of ethyl acetate, further twice with 40 mL of dichloromethane and then twice with 50 mL of dichloromethane. The joined extracted layers were dried by magnesium sulfate anhydride and then concentrated under reduced pressure to dryness to obtain 4.9 g of 4-methylthio-3-methylpyridine-1-oxide in solid form.

### (2) Production of 4-methylthio-2-keto-3-methylpyridine

68.0 g (666 mmol, 22 equivalent) of acetic acid anhydride was added to 4.7 g (30.3 mmol, 1.0 equivalent) of 4-methylthio-3-methylpyridine-1-oxide to react at 107 to 115°C for 20 hr. 63 mL of ethyl acetate and 8.4 mL of methanol were added to the concentrated residue obtained after removing acetic acid anhydride by distillation, which was further refluxed with stirring for 3 hr. The reaction solution was cooled and distilled at 40°C or less under reduced pressure to remove a solvent. The obtained concentrated residue was refined by silica gel column chromatography to obtain 3.62 g of 4-methylthio-2-keto-3-methylpyridine as an oily substance.

### (3) Production of 4-methylthio-3-methylpyridine-2-thione

4.8 g (11.9 mmol, 1.1 equivalent) of a Lawesson's reagent and 38 mL of toluene were added to 3.4 g (21.9 mmol, 1.0 equivalent) of 4-methylthio-2-keto-3-methylpyridine and the mixture was stirred under heating/refluxing for 11 hr. After the reaction solution was cooled, it was distilled at 40°C or less under reduced pressure to remove a solvent and the obtained concentrated residue was refined by silica gel column chromatography to thereby obtain 1.33 g of 4-methylthio-3-methylpyridine-2-thione as a solid.

### (4) Production of 2-[[(4-thiomethyl-3-methyl-2-pyridyl)thio]-methyl]-1H-benzi midazole hydrochloride

1.29 g (7.5 mmol, 1.0 equivalent) of
4-methylthio-3-methylpyridine-2-thione and 1.38 g (8.3 mmol, 1.1 equivalent) of 2- (chloromethyl)benzimidazole were added in a mixed solution of 1.10 g of an aqueous 30% sodium hydroxide solution and 100 mL of ethanol, and the mixture was stirred at 50°C for 2 hr. Because the reaction was not completed, 250 mg (1.5 mmol, 0.20 equivalent) of 2-(chloromethyl)benzimidazole was further added to the reaction solution, which was then stirred at 50°C for 1 hr. 0.20 g of an aqueous 30% sodium hydroxide solution was further added to the reaction solution, which was further reacted at 50°C for 30 min. After the reaction solution was concentrated at 40°C or less under reduced pressure, 94 mL of water was added to the solution, which was then extracted with 300 mL of dichloromethane and then with 100 mL of dichloromethane. After the organic phase was dried by magnesium sulfate anhydride, the solvent was concentrated to dryness. The obtained residue was refined by silica gel column chromatography. A residue obtained by concentrating the target fraction after the chromatography was crystallized from ethanolic hydrochloric acid solution to obtain 62 mg of a colorless crystal of the target
2-[[(4-thiomethyl-3-methyl-2-pyridyl)thio]-methyl]-1H-benzi midazole hydrochloride.
The total yield was 0.62% and the purity measured by HPLC was 97.8%.

¹H-NMR (400 MHz, CDCl₃) δ:
2.22 (3H, s), 4.91 (2H, s), 7.04 (1H, d J = 5.6 Hz), 7.50-7.54 (2H, m), 7.72-7.77 (2H, m), 8.14 (1H, d J = 5.6 Hz) MS m/z : 301 (M⁺: free body)

### Example 6

### Production of 2-[[(4-thiopropyl-3-methyl-2-pyridyl)thio]-methyl]-1H-benzi midazole hydrochloride

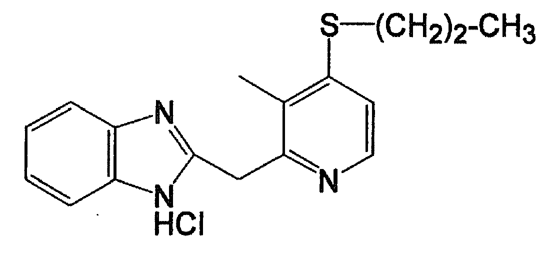

### (1) Production of 4-propylthio-3-methylpyridine-1-oxide

5.0 g (34.8 mmol, 1.0 equivalent) of
4-chloro-3-methylpyridine-1-oxide, 7.0 g (52.5 mmol, 1.5 equivalent) of an aqueous 30% sodium hydroxide solution and 2.7 g (35.5 mmol, 1.0 equivalent) of 1-propanethiol were added to 74 mL of ethanol, and the mixture was reacted at 72 to 73°C under heating/stirring for 1 hr and 30 min. Because the reaction was not completed, 0.5 g (6.6 mmol, 0.2 equivalent) of
1-propanethiol and 1.4 g (10.5 mmol, 0.3 equivalent) of an aqueous 30% sodium hydroxide solution were further added to the reaction solution, which was then further reacted at 2 to 73°C under heating/stirring for 1 hr and 30 min. After cooled to 30°C, the reaction solution was distilled under reduced pressure to remove a solvent. After 20 mL of water was added to the concentrated residue, the residue was extracted four times in total, that is, twice with 37 mL of ethyl acetate and twice with 30 mL of ethyl acetate. The extract was dried by magnesium sulfate anhydride, and then concentrated under reduced pressure to dryness to thereby obtain 6.25 g of
4-propylthio-3-methylpyridine-1-oxide as a solid.

### (2) Production of 4-propylthio-2-keto-3-methylpyridine

40.8 g (730 mmol, 22 equivalent) of acetic acid anhydride was added to 6.05 g (33.0 mmol, 1.0 equivalent) of 4-propylthio-3-methylpyridine-1-oxide to react at 108 to 110°C for 18 hr. 69 mL of ethyl acetate and 9.5 mL of methanol were added to the concentrated residue obtained after removing acetic acid anhydride by distillation, which was further refluxed with stirring for 2 hr. The reaction solution was cooled and distilled at 40°C or less under reduced pressure to remove a solvent. The obtained concentrated residue was refined by silica gel column chromatography to obtain 3.5 g of 4-propylthio-2-keto-3-methylpyridine as an oily substance.

### (3) Production of 4-propylthio-3-methylpyridine-2-thione

0.83 g (11.9 mmol, 1.1 equivalent) of a Lawesson's reagent and 6.6 mL of toluene were added to 0.7 g (3.8 mmol, 1.0 equivalent) of 4-propylthio-2-keto-3-methylpyridine and the mixture was stirred under heating/refluxing for 11 hr. After the reaction solution was cooled, it was distilled at 40°C or less under reduced pressure to remove a solvent and the obtained concentrated residue was refined by silica gel column chromatography. 2 mL of 1-hexane was then added to the refined residue to wash the residue three times to obtain 0.081 g of 4-propylthio-3-methylpyridine-2-thione as a solid.
The target material was produced in a larger amount in the same manner as above.
3.3 g (8.2 mmol, 1.1 equivalent) of a Lawesson's reagent and 26 mL of toluene were added to 2.8 g (15.3 mmol, 1.0 equivalent) of 4-propylthio-2-keto-3-methylpyridine and the mixture was stirred under heating/refluxing for 7 hr and 30 min. After the reaction solution was cooled, it was distilled at 40°C or less under reduced pressure to remove a solvent and 50 mL of dichloromethane was added to the obtained residue to remove an insoluble substance by filtration. A concentrated residue obtained by concentrating the filtrate under reduced pressure was refined by silica gel column chromatography to obtain 0.29 g of 4-propylthio-3-methylpyridine-2-thione as a solid.

### (4) Production of 2-[[(4-thiopropyl-3-methyl-2-pyridyl)thio]-methyl]-1H-benzi midazole hydrochloride

370 mg (1.86 mmol, 1.0 equivalent) of 4-thiopropyl-3-methylpyridine-2-thione and 340 mg (2.0 mmol, 1.1 equivalent) of 2- (chloromethyl) benzimidazole were added in a mixed solution of 320 mg of an aqueous 30% sodium hydroxide solution and 24 mL of ethanol, and the mixture was stirred at 50°C for 2 hr. Because the reaction was not completed, 62 mg (0.37 mmol, 0.5 equivalent) of 2-(chloromethyl)benzimidazole and 50 mg of an aqueous 30% sodium hydroxide solution were further added to the reaction solution, which was further reacted at 50°C for 3 hr. After the reaction solution was concentrated at 40°C or less under reduced pressure, 39 mL of water was added to the solution, which was then extracted with 100 mL of dichloromethane and then with 68 mL of dichloromethane. After the organic phase was dried by magnesium sulfate anhydride, the solvent was concentrated to dryness. The obtained residue was refined by silica gel column chromatography. A residue obtained by concentrating the target fraction after the chromatography was crystallized from ethanolic hydrochloric acid solution to obtain 93 mg of a colorless crystal of 2-[[(4-propoxy-3-methyl-2-pyridyl)thio]-methyl]-1H-benzimid azole hydrochloride.
The total yield was 0.79% and the purity measured by HPLC was 99.5%.

¹H-NMR (400 MHz, DMSO-d₆) δ:
0.99 (3H, t J = 7.2 Hz), 1.61-1.66 (2H, m), 2.22 (3H, s), 3.02 (2H, t J = 7.2 Hz), 4.92 (2H, s), 7.09 (1H, d J = 5.6 Hz), 7.50-7.55 (2H, m), 7.73-7.78 (2H, m), 8.12 (1H, d J = 5.6 Hz) MS m/z : 329 (M⁺: free body)

### Example 7

### Production of 2-[[(4-thiopentyl-3-methyl-2-pyridyl)thio]-methyl]-1H-benzi midazole

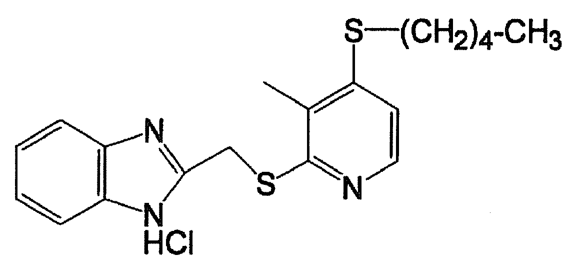

### (1) Production of 4-pentylthio-3-methylpyridine-1-oxide

A mixture of 3.63 g (34.8 mmol, 1.0 equivalent) of 1-pentane thiol, 74 mL of ethanol, and 7.0 g (52.5 mmol, 1.5 equivalent) of an aqueous 30% sodium hydroxide solution was stirred under heating/refluxing for 1 hr. After the reaction mixture was cooled to 30°C, 5.0 g (34.8 mmol, 1.0 equivalent) of 4-chloro-3-methylpyridine-1-oxide was added to the reaction solution, which was again stirred under heating for 1 hr. After cooled to ambient temperature, the reaction solution was distilled under reduced pressure to remove a solvent. After 20 mL of water was added to the concentrated residue, the residue was extracted twice in total, that is, once with 20 mL of ethyl acetate and once with 37 mL of ethyl acetate. The extract was dried by magnesium sulfate anhydride, and then concentrated under reduced pressure to dryness to thereby obtain 6.0 g of 4-pentylthio-3-methylpyridine-1-oxide as an oily substance.

### (2) Production of 4-pentylthio-2-keto-3-methylpyridine

78.2 g (766 mmol, 21 equivalent) of acetic acid anhydride was added to 7.8 g (36.9 mmol, 1.0 equivalent) of 4-pentylthio-3-methylpyridine-1-oxide to react at 108 to 111°C for 15 hr. 78 mL of ethyl acetate and 10.4 mL of methanol were added to the concentrated residue obtained after removing acetic acid anhydride by distillation, which was further refluxed with stirring for 3 hr. The reaction solution was cooled and then distilled at 40°C or less under reduced pressure to remove a solvent. The obtained concentrated residue was refined by silica gel column chromatography to obtain 2.61 g of 4-pentylthio-2-keto-3-methylpyridine as an oily substance.

### (3) Production of 4-pentylthio-3-methylpyridine-2-thione

0.56 g (1.4 mmol, 1.1 equivalent) of a Lawesson's reagent and 5 mL of toluene were added to 0.54 g (2.6 mmol, 1.0 equivalent) of 4-pentylthio-2-keto-3-methylpyridine and the mixture was stirred under heating/refluxing for 9 hr. After the reaction solution was cooled, it was distilled at 40°C or less under reduced pressure to remove a solvent and the obtained concentrated residue was refined by silica gel column chromatography to thereby obtain 0.343 g of 4-pentylthio-3-methylpyridine-2-thione as solid powders.

### (4) Production of 2-[[(4-thiopentyl-3-methyl-2-pyridyl)thio]-methyl]-1H-Benzi midazole

330 mg (1.7 mmol, 1.0 equivalent) of 4-thiopentyl-3-methylpyridine-2-thione and 304 mg (1.8 mmol, 1.1 equivalent) of 2 - (chloromethyl) benzimidazole were added in a mixed solution of 243 mg of an aqueous 30% sodium hydroxide solution and 13 mL of ethanol, and the mixture was stirred at 50°C for 2 hr. After the reaction solution was concentrated at 40°C or less under reduced pressure, 21 mL of water was added to the solution, which was then extracted with 91 mL of dichloromethane. After the organic phase was dried by magnesium sulfate anhydride, the solvent was concentrated to dryness. The obtained residue was refined by silica gel column chromatography. A residue obtained by concentrating the target fraction after the chromatography was concentrated and refined to obtain 222 mg of a colorless crystal of the target 2-[[(4-thiopentyl-3-methyl-2-pyridyl)thio]methyl]-1H-benzim idazole.
The total yield was 6.8% and the purity measured by HPLC was 99.6%.

¹H-NMR (400 MHz, CDCl₃) δ:
0.93 (3H, t J = 7.2 Hz), 1.35-1.51 (4H, m), 1.71-1.78 (2H, m), 2.25 (3H, s), 2.97 (2H, t J = 7.6 Hz), 4.57 (2H, s), 6.96 (1H, d J = 5.6 Hz), 7.18-7.23 (2H, m), 7.35 (1H, bs), 7.72 (1H, bs), 8.33 (1H, d J = 5.6 Hz), 10.90 (1H, bs)
MS m/z : 357 (M⁺: free body)

### Example 8

### Production of 2-[[4-[[2-[2-(2,2,2-trifluoroethoxy)ethoxy]-ethyl]thio]-3-m ethyl-2-pyridyl]thio]-methyl]-1H-benzimidazole hydrochloride

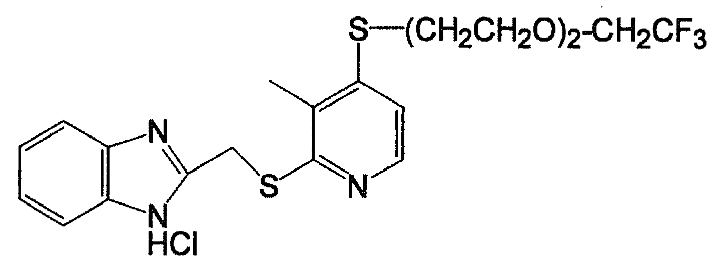

### (1) Production of 4-[2-(2-(2,2,2-trifluoroethoxy)-ethoxy)ethyl]thio-3-methylp yridine-1-oxide

8.71 g (217.8 mmol, 1.5 equivalent) of sodium hydrochloride, 8.71 mL of water, 31.18 g (150.9 mmol, 1.1 equivalent) of
1-chloro-2-[2-(2,2,2-trifluoroethoxy)ethoxy]ethane and 300 mL of ethanol were added to 20.02 g (141.8 mmol, 1. 0 equivalent) of 4-mercapto-3-methylpyridine-1-oxide produced in Reference Example 1, and the mixture was refluxed with stirring for 8 hr, and then the reaction was stopped. After the reaction solution was cooled, it was distilled at 40°C or less under reduced pressure to remove a solvent and 80 mL of water was added to the obtained residue, which was then extracted with 300 mL of ethyl acetate. The obtained organic phase was dried by magnesium sulfate anhydride, and then the dried organic phase was distilled at 40°C or less under reduced pressure to remove a solvent to thereby obtain 46.01 g of 4-[2-(2-(2,2,2-trifluoroethoxy)-ethoxy)ethyl]thio-3-methylp yridine-1-oxide as an oily substance.

### (2) Production of 4-[2-(2-(2,2,2-trifluoroethoxy)-ethoxy)ethyl]thio-2-keto-3-methylpyridine

324 g (3.2 mol, 22 equivalent) of acetic acid anhydride was added to 45.73 g (146.9 mmol, 1.0 equivalent) of
4-[2-(2-(2,2,2-trifluoroethoxy)-ethoxy)ethyl]thio-3-methylp yridine-1-oxide to react at 100°C for 15 hr. 300 mL of ethyl acetate and 40 mL of methanol were added to the concentrated residue obtained after acetic acid anhydride was removed by distillation and the obtained mixture was further refluxed with stirring for 2 hr. After the reaction solution was cooled, it was distilled at 40°C or less under reduced pressure to remove a solvent. The obtained concentrated residue was refined by silica gel column chromatography to obtain 18.50 g of
4-[2-(2-(2,2,2-trifluoroethoxy)-ethoxy)ethyl]thio-2-keto-3-methylpyridine as an oily substance.

### (3) Production of 4-[2-(2-(2,2,2-trifluoroethoxy)-ethoxy)ethyl]thio-3-methylp yridine-2-thione

12.57 g (31.1 mmol, 1.1 equivalent) of a Lawesson's reagent and 100 mL of toluene were added to 18.03 g (57.9 mmol, 1.0 equivalent) of
4-[2-(2-(2,2,2-trifluoroethoxy)-ethoxy)ethyl]thio-2-keto-3-methylpyridine and the mixture was stirred under heating/refluxing for 8 hr. The reaction mixture was cooled and distilled at 40°C or less under reduced pressure to remove a solvent. The concentrated residue was refined by silica gel chromatography to obtain 4.90 g of
4-[2-(2-(2,2,2-trifluoroethoxy)-ethoxy)ethyl]thio-3-methylpyridine-2-thione as brown powders.

### (4) Production of 2-[[4-[[2-[2-(2,2,2-trifluoroethoxy)ethoxy]-ethyl]thio]-3-m ethyl-2-pyridyl]thio]methyl]-1H-benzimidazole hydrochloride

4.08 g (12.5 mol, 1.0 equivalent) of
4-[2-(2-(2,2,2-trifluoroethoxy)-ethoxy)ethyl]thio-3-methylp yridine-2-thione and 2.20 g (13.2 mmol, 1.1 equivalent) of 2-(chloromethyl)benzimidazole were added in a mixed solution of 570 mg of an aqueous 30% sodium hydroxide solution and 50 mL of ethanol, and the mixture was stirred at 50°C for 3 hr. After the reaction solution was concentrated at 40°C or less under reduced pressure, 100 mL of water was added to the solution, which was then extracted with 300 mL of dichloromethane. After the organic phase was dried by magnesium sulfate anhydride, the solvent was concentrated to dryness. The obtained residue was refined by silica gel column chromatography. A residue obtained by concentrating the target fraction after the chromatography was crystallized from ethanolic hydrochloric acid solution to obtain 4.63 g of a colorless crystal of the target
2-[[4-[[2-[2-(2,2,2-trifluoroethoxy)ethoxy]-ethyl]thio]-3-m ethyl-2-pyridyl]thio]-methyl]-1H-benzimidazole hydrochloride.
The total yield was 11.0% and the purity measured by HPLC was 99.1%.

¹H-NMR (400 MHz, DMSO-d₆)δ:
2.22 (3H, s), 3.26 (2H, t J = 6.2 Hz), 3.50-3.60 (2H, m), 3.66 (2H, t J = 6.3 Hz), 3.69-3.71 (2H, m), 4.06 (2H, q J = 9.3 Hz), 4.94 (2H, s), 7.14 (1H, d J = 5.1 Hz), 7.50-7.55 (2H, m), 7.74-7.80 (2H, m), 8.14 (1H, d J = 5.2 Hz), 15.2 (1H, bs)
MS m/z : 457 (M⁺: free body)

### Example 9

### Production of 2-[[4-[2-[2-(2,2,2-trifluoroethoxy)-ethoxy]-ethoxy]-3-methy 1-2-pyridyl]thio]-methyl]-1H-benzimidazole hydrochloride

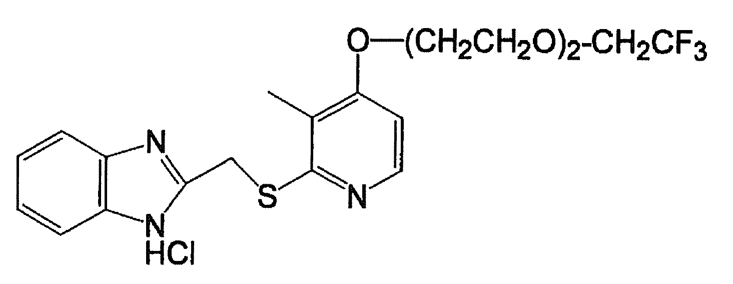

### (1) Production of 4-[2-(2-(2,2,2-trifluoroethoxy)-ethoxy)-ethoxy]-3-methylpyr idine-1-oxide

17.01 g (118.5 mmol, 1.0 equivalent) of 4-chloro-3-methylpyridine-1-oxide and 9.77 g (244 mmol, 2.1 equivalent) of sodium hydroxide were added to 44.58 g (237 mmol, 2.0 equivalent) of
2-[2-(2,2,2-trifluoroethoxy)-ethoxy]-ethanol and 80 mL of toluene, and the mixture was refluxed with stirring for 6 hr, and then the reaction was stopped. After the reaction solution was cooled, 140 mL of water and 11.84 g of concentrated hydrochloric acid were added to the reaction solution, which was then extracted with 280 mL of ethyl acetate. The organic phase was dried by magnesium sulfate anhydride, and then the dried organic phase was dried at 40°C or less under reduced pressure to remove a solvent to thereby obtain 48.90 g of 4-[2-(2-(2,2,2-trifluoroethoxy)-ethoxy)-ethoxy]-3-methylpyr idine-1-oxide as an oily substance.

### (2) Production of 4-[2-(2-(2,2,2-trifluoroethoxy)-ethoxy)ethoxy]-2-keto-3-met hylpyridine

324 g (3.2 mol, 19 equivalent) of acetic acid anhydride was added to 48.69 g (164.9 mmol, 1.0 equivalent) of
4-[2-(2-(2,2,2-trifluoroethoxy)-ethoxy)-ethoxy]-3-methylpyr idine-1-oxide to react at 100°C for 11 hr. 300 mL of ethyl acetate and 40 mL of methanol were added to the concentrated residue obtained after acetic acid anhydride was removed by distillation and the obtained mixture was refluxed with stirring for 30 min. After the reaction solution was cooled, it was distilled at 40°C or less under reduced pressure to remove a solvent. The obtained concentrated residue was refined by silica gel column chromatography to obtain 8.70 g of
4-[2-(2-(2,2,2-trifluoroethoxy)-ethoxy)ethoxy]-2-keto-3-met hylpyridine as an oily substance.

### (3) Production of 4-[2-(2-(2,2,2-trifluoroethoxy)-ethoxy)ethoxy]thio-3-methyl pyridine-2-thione

6.25 g (16.8 mmol, 1.2 equivalent) of a Lawesson's reagent and 50 mL of toluene were added to 8.60 g (29.1 mmol, 1.0 equivalent) of
4-[2-(2-(2,2,2-trifluoroethoxy)-ethoxy)ethoxy]-2-keto-3-met hylpyridine and the mixture was stirred under heating/refluxing for 11 hr. After the reaction mixture was cooled to 30°C, 6.15 g (15.2 mmol, 1.0 equivalent) of a Lawesson' s reagent was added to the reaction solution, which was further reacted under heating/refluxing for 11 hr. The reaction solution was cooled and distilled at 40°C or less under reduced pressure to remove a solvent and the concentrated residue was refined by silica gel column chromatography to obtain 1.40 g of 4-[2-(2-(2,2,2-trifluoroethoxy)-ethoxy)ethoxy]thio-3-methyl pyridine-2-thione as powders.

### (4) Production of 2-[[4-[2-[2-(2,2,2-trifluoroethoxy)-ethoxy]-ethoxy]-3-methy 1-2-pyridyl-thio]-methyl]-1H-benzimidazole hydrochloride

4.08 g (12.5 mol, 1.0 equivalent) of
4-[2-[2-(2,2,2-trifluoroethoxy)-ethoxy]-ethoxy]-3-methylpyr idine-2-thione and 2.20 g (13.2 mmol, 1.1 equivalent) of 2- (chloromethyl) benzimidazole were added in a mixed solution of 570 mg of an aqueous 30% sodium hydroxide solution and 50 mL of ethanol, and the mixture was stirred at 50°C for 3 hr. After the reaction solution was concentrated at 40°C or less under reduced pressure, 100 mL of water was added to the solution, which was then extracted with 300 mL of dichloromethane. After the organic phase was dried by magnesium sulfate anhydride, the solvent was concentrated to dryness. The obtained residue was refined by silica gel column chromatography. A residue obtained by concentrating the target fraction after the chromatography was crystallized from ethanolic hydrochloric acid solution to obtain 4.63 g of a colorless crystal of the target
2-[[4-[2-[2-(2,2,2-trifluoroethoxy)-ethoxy]-ethoxy]-3-methy 1-2-pyridyl-thio]-methyl]-1H-benzimidazole hydrochloride.
The total yield was 3.0% and the purity measured by HPLC was 99.1%.

¹H-NMR (400 MHz, DMSO-d₆) δ:
2.12 (3H, s), 3.62-3.66 (2H, m), 3.70-3.74 (2H, m), 3.76-3.80 (2H, m), 4.07 (2H, q J = 9.3 Hz), 4.19-4.23 (2H, m), 4.94 (2H, s), 6.92 (1H, d, J = 5.9 Hz), 7.50-7.56 (2H, m), 7.74-7.79 (2H, m), 8.19 (1H, t J = 5.9 Hz), 15.2 (1H, bs) MS m/z : 457 (M⁺: free body)

### Example 10

### Production of 2-[[(4-pentyloxy-3-methyl-2-pyridyl)thio]-methyl]-1H-benzim idazole hydrochloride

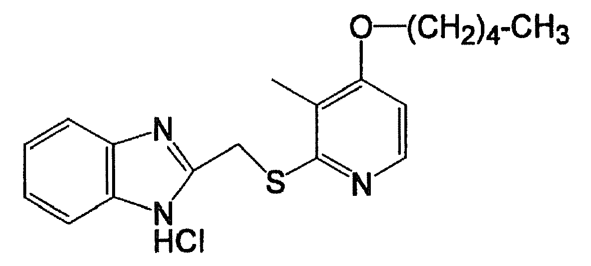

### (1) Production of 4-pentyloxy-3-methylpyridine-1-oxide

12.04 g (83.9 mmol, 1.0 equivalent) of 4-chloro-3-methylpyridine-1-oxide and 6.79 g (170 mmol, 2.0 equivalent) of sodium hydroxide were added to 14.76 g (167 mmol, 2.0 equivalent) of 1-pentanol and 58 mL of toluene and the mixture was refluxed with stirring for 5 hr, and then the reaction was stopped. After the reaction solution was cooled, 97 mL of water and 7.24 g of concentrated hydrochloric acid were added to the reaction solution, which was then extracted with 98 mL of ethyl acetate. The organic phase was dried by magnesium sulfate anhydride, and then the dried organic phase was dried at 40°C or less under reduced pressure to remove a solvent to thereby obtain 17.56 g of
4-pentyloxy-3-methylpyridine-1-oxide as an oily substance.

### (2) Production of 4-pentyloxy-2-keto-3-methylpyridine

248 g (2.43 mol, 27 equivalent) of acetic acid anhydride was added to 17.56 g (89.9 mmol, 1.0 equivalent) of 4-pentyloxy-3-methylpyridine-1-oxide to react at 105°C to 110°C for 9 hr. 230 mL of ethyl acetate and 34.5 mL of methanol were added to the concentrated residue obtained after acetic acid anhydride was removed by distillation and the obtained mixture was refluxed with stirring for 8 hr. After the reaction solution was cooled, it was distilled at 40°C or less under reduced pressure to remove a solvent. The obtained concentrated residue was refined by silica gel column chromatography to obtain 8.70 g of
4-pentyloxy-2-keto-3-methylpyridine as an oily substance.

### (3) Production of 4-pentyloxy-3-methylpyridine-2-thione

6.18 g (15.3 mmol, 1.0 equivalent) of a Lawesson's reagent and 52 mL of toluene were added to 5.8 g (29.7 mmol, 1.0 equivalent) of 4-pentyloxy-2-keto-3-methylpyridine and the mixture was stirred under heating/refluxing for 11 hr and 30 min. After the reaction mixture was cooled to 30°C, 6. 18 g (15.3 mmol, 1.0 equivalent) of a Lawesson's reagent was added to the reaction solution, which was further reacted under heating/refluxing for 16 hr and 30 min. The reaction solution was cooled and distilled at 40°C or less under reduced pressure to remove a solvent and the concentrated residue was refined by silica gel column chromatography to obtain 0.338 g of 4-pentyloxy-3-methylpyridine-2-thione as powders.

### (4) Production of 2-[[(4-pentyloxy-3-methyl-2-pyridyl)thio]-methyl]-1H-benzim idazole hydrochloride

338 mg (1.6 mmol, 1.0 equivalent) of 4-pentyloxy-3-methylpyridine-2-thione and 362 mg (2.2 mmol, 1.1 equivalent) of 2- (chloromethyl) benzimidazole were added in a mixed solution of 0.290 mL of an aqueous 30% sodium hydroxide solution and 12 mL of ethanol, and the mixture was stirred at 50°C for 4 hr and 30 min. 19 mL of water was added to the reaction solution, which was then extracted with 84 mL of dichloromethane and then with 20 mL of dichloromethane. After the organic phase was dried by magnesium sulfate anhydride, the solvent was concentrated to dryness. The obtained residue was refined by silica gel column chromatography. A residue obtained by concentrating the target fraction after the chromatography was crystallized from ethanolic hydrochloric acid solution to obtain 134.8 mg of a colorless crystal of the target 2-[[(4-pentyloxy-3-methyl-2-pyridyl)thio]methyl]-1H-benzimi dazole hydrochloride.
The total yield was 0.64% and the purity measured by HPLC was 97.4%.

¹H-NMR (400 MHz, DMSO-d₆) δ:
0.89 (3H, t J = 7.6 Hz), 1.29-1.42 (4H, m), 1.73 (2H, m J = 6.8 Hz), 2.11 (3H, s), 4.06 (2H, t J = 6.4 Hz), 4.93 (2H, s), 6.89 (1H, d J = 6.0 Hz), 7.50-7.55 (2H, m), 7.74-7.79 (2H, m), 8.17 (1H, d J = 5.6 Hz)
MS m/z : 341 (M⁺: free body)

### Example 11

### Production of 2-[[4-(3-ethoxy-1-propoxy)-3-methyl-2-pyridyl]thio]-methyl] -1H-benzimidazole

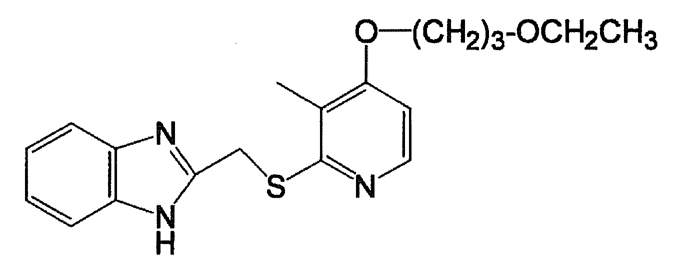

### (1) Production of 4-[(3-ethoxy-1-propyl)oxy]-3-methylpyridine-1-oxide

3.52 g (24.5 mmol, 1.0 equivalent) of 4-chloro-3-methylpyridine-1-oxide and 2.0 g (50 mmol, 2.0 equivalent) of sodium hydroxide were added to 4.80 g (46.1 mmol, 1.9 equivalent) of 3-ethoxy-1-propanol and 17 mL of toluene and the mixture was refluxed with stirring for 4 hr, and then the reaction was stopped. After the reaction solution was cooled, 30 mL of water and 1.7 g of concentrated hydrochloric acid were added to the reaction solution, which was then extracted twice with 50 mL of ethyl acetate and twice with 30 mL of dichloromethane. The organic phase was dried by magnesium sulfate anhydride, and then the dried organic phase was dried at 40°C or less under reduced pressure to remove a solvent to thereby obtain 5.6 g of
4-[(3-ethoxy-1-propyl)oxy]-3-methylpyridine-1-oxide as an oily substance.

### (2) Production of 4-[(3-ethoxy-1-propyl)oxy]-2-keto-3-methylpyridine

64.9 g (636 mmol, 24 equivalent) of acetic acid anhydride was added to 5.5 g (26.0 mmol, 1.0 equivalent) of 4-[(3-ethoxy-1-propyl)oxy]-3-methylpyridine-1-oxide to react at 118°C for 4 hr. 60 mL of ethyl acetate and 9 mL of methanol were added to the concentrated residue obtained after acetic acid anhydride was removed by distillation and the obtained mixture was further refluxed with stirring for 2 hr. After the reaction solution was cooled, it was distilled at 40°C or less under reduced pressure to remove a solvent. The obtained concentrated residue was refined by silica gel column chromatography to obtain 1.74 g of
4-[(3-ethoxy-1-propyl)oxy]-2-keto-3-methylpyridine as an oily substance.

### (3) Production of 4-[(3-ethoxy-1-propyl)oxy]-3-methylpyridine-2-thione

1.60 g (4.0 mmol, 1.0 equivalent) of a Lawesson's reagent and 13 mL of toluene were added to 1.67 g (7.9 mmol, 1.0 equivalent) of
4-[(3-ethoxy-1-propyl)oxy]-2-keto-3-methylpyridine and the mixture was stirred under heating/refluxing for 14 hr and 30 min. After the reaction mixture was cooled to 30°C, 1. 54 g (3.8 8 mmol, 1.0 equivalent) of a Lawesson's reagent was added to the reaction solution, which was further reacted under heating/refluxing for 10 hr. The reaction solution was cooled and distilled at 40°C or less under reduced pressure to remove a solvent and the concentrated residue was refined by silica gel column chromatography to obtain 0.44 g of 4-[(3-ethoxy-1-propyl)oxy]-3-methylpyridine-2-thione as powders.

### (4) Production of 2-[[4-(3-ethoxy-1-propoxy)-3-methyl-2-pyridyl]thio]-methyl] -1H-benzimidazol

420 mg (1.8 mmol, 1.0 equivalent) of 4-[(3-ethoxy-1-propyl)oxy]-3-methylpyridine-2-thione and 350 mg (2.1 mmol, 1.1 equivalent) of 2- (chloromethyl) benzimidazole were added in a mixed solution of 276 mg of an aqueous 30% sodium hydroxide solution and 14 mL of ethanol, and the mixture was stirred at 50°C for 3 hr. After the reaction solution was concentrated at 40°C or less under reduced pressure, 23 mL of water was added to the reaction solution, which was then extracted with dichloromethane (100 mL, 30 mL x 2) sequentially. After the organic phase was dried by magnesium sulfate anhydride, the solvent was concentrated to dryness. The obtained residue was refined by silica gel column chromatography to thereby obtain 220 mg of the target
2-[[4-(3-ethoxy-1-propoxy)-3-methyl-2-pyridyl]thio]methyl]-1H-benzimidazole.
The total yield was 2.9% and the purity measured by HPLC was 98.6%.

¹H-NMR (400 MHz, DMSO-d₆) δ:
1.10 (3H, t J = 7.2 Hz), 1.93-1.99 (2H, m), 2.07 (3H, s), 3.42 (2H, q J = 7.2 Hz), 3.51 (2H, t J = 6.4 Hz), 4.20 (2H, t J = 6.0 Hz), 4.66 (2H, s), 6.88 (1H, d J = 6.0 Hz), 7.12-7.15 (2H, m), 7.42-7.54 (2H, m), 8.27 (1H, d J = 6.0 Hz)
MS m/z : 357 (M⁺)

### Example 12

### Production of 2-[[[4-[2-(2-methoxyethoxy)-ethoxy]-3-methyl-2-pyridyl]thio ]-methyl]-1H-benzimidazole hydrochloride

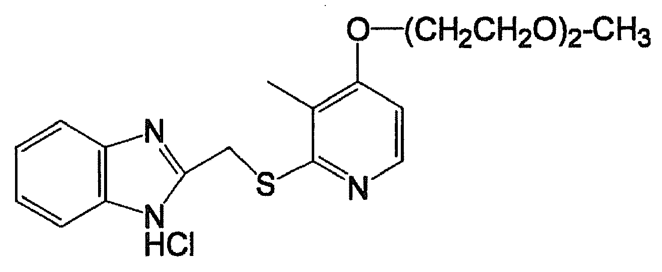

### (1) Production of 4-[2-(2-methoxyethoxy)ethoxy]-3-methylpyridine-1-oxide

3.50 g (24.4 mmol, 1.0 equivalent) of 4-chloro-3-methylpyridine-1-oxide and 1.97 g (49.3 mmol, 2.0 equivalent) of sodium hydroxide were added to 5.86 g (48.8 mmol, 2.0 equivalent) of 2-(2-methoxyethoxy)ethanol and 16 mL of toluene and the mixture was refluxed with stirring for 5 hr, and then the reaction was stopped. After the reaction solution was cooled, 28 mL of water and 0.90 g of concentrated hydrochloric acid were added to the reaction solution, which was then extracted with 31 mL of ethyl acetate and further with 60 mL (x 2) of dichloromethane. The organic phase was dried by magnesium sulfate anhydride, and then the dried organic phase was dried at 40°C or less under reduced pressure to thereby obtain 5.91 g of
4-[2-(2-methoxyethoxy)ethoxy]-3-methylpyridine-1-oxide as an oily substance.

### (2) Production of 4-[2-(2-methoxyethoxy)ethoxy]-2-keto-3-methylpyridine

75. 6 g (741 mmol, 168 equivalent) of acetic acid anhydride was added to 5.84 g (25.7 mmol, 1.0 equivalent) of 4-[2-(2-methoxyethoxy)ethoxyl-3-methylpyridine-1-oxide to react at 100°C to 113°C for 5 hr. 70 mL of ethyl acetate and 15 mL of methanol were added to the concentrated residue obtained after acetic acid anhydride was removed by distillation and the obtained mixture was further refluxed with stirring for 3 hr. After the reaction solution was cooled, it was distilled at 40°C or less under reduced pressure to remove a solvent. The obtained concentrated residue was refined by silica gel column chromatography to obtain 1.35 g of 4-[2-(2-methoxyethoxy)ethoxyl-2-keto-3-methylpyridine as an oily substance.

### (3) Production of 4-[2-(2-methoxyethoxy)ethoxy]-3-methylpyridine-2-thione

1.30 g (3.2 mmol, 1.1 equivalent) of a Lawesson' s reagent and 11 mL of toluene were added to 1.33 g (5.9 mmol, 1.0 equivalent) of
4-[2-(2-methoxyethoxy)ethoxy]-2-keto-3-methylpyridine and the mixture was stirred under heating/refluxing for 12 hr. After the reaction mixture was cooled to 30°C, 1.32 g (3.3 mmol, 1.1 equivalent) of a Lawesson's reagent was added to the reaction solution, which was further reacted under heating/refluxing for 10 hr. The reaction solution was cooled and distilled at 40°C or less under reduced pressure to remove a solvent and the concentrated residue was refined by silica gel column chromatography to obtain 87.1 mg of
4-[2-(2-methoxyethoxy)ethoxy]-3-methylpyridine-2-thione as powders.

### (4) Production of 2-[[[4-[2-(2-methoxyethoxy)ethoxy]-3-methyl-2-pyridyl]thio] -methyl]-1H-benzimidazole hydrochloride

80.9 mg (0.33 mmol, 1.0 equivalent) of 4-[2-(2-methoxyethoxy)ethoxy]-3-methylpyridine-2-thione and 63.5 mg (0.38 mmol, 1.2 equivalent) of
2-(chloromethyl)benzimidazole were added in a mixed solution of 0.051 mL of an aqueous 30% sodium hydroxide solution and 3 mL of ethanol, and the mixture was stirred at 50°C for 3 hr and 30 min. 6 mL of water was added to the reaction solution, which was then extracted with dichloromethane (once with 30 mL thereof and twice with 12 mL thereof) sequentially. After the organic phase was dried by magnesium sulfate anhydride, the solvent was concentrated to dryness. The obtained residue was refined by silica gel column chromatography. A residue obtained by concentrating the target fraction after the chromatography was crystallized from ethanolic hydrochloric acid solution to obtain 77.5 mg of a colorless crystal of the target 2-[[[4-[2-(2-methoxyethoxy)ethoxy]-3-methyl-2-pyridyl)thio] -methyl]-1H-benzimidazole hydrochloride.
The total yield was 0.87% and the purity measured by HPLC was 98.2%.

¹H-NMR (400 MHz, DMSO-d₆) δ:
2.12 (3H, s), 3.44 (2H, t J = 4.8 Hz), 3.59 (2H, t J = 4.8 Hz), 3.75 (2H, t J = 4.4 Hz), 4.18 (2H, t J = 4.4 Hz), 4.92 (2H, s), 6.89 (1H, d J = 6.0 Hz), 7.51-7.55 (2H, m), 7.74-7.78 (2H,m), 8.15 (1H, d J = 5.6 Hz)
MS m/z : 373 (M⁺: free body)

### Example 13

### Production of 2-[[[4-(8-hydroxy-1-octyloxy)-3-methyl-2-pyridyl]thio]-meth yl]-1H-benzimidazole

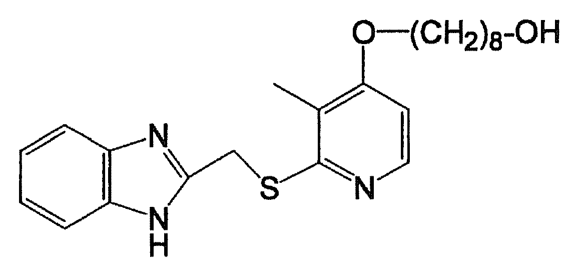

### (1) Production of 4-[(8-hydroxy-1-octyl)oxy]-3-methylpyridine-1-oxide

10.00 g (69.7 mmol, 1.0 equivalent) of 4-chloro-3-methylpyridine-1-oxide and 5.60 g (140 mmol, 2.0 equivalent) of sodium hydroxide were added to 20.5 g (140 mmol, 2.0 equivalent) of 1,8-octanediol and 46 mL of toluene and the mixture was refluxed with stirring for 5 hr and 30 min, and then the reaction was stopped. After the reaction solution was cooled, 80 mL of water and 5.0 g of concentrated hydrochloric acid were added to the reaction solution, and then 1.8 g of an aqueous 30% sodium hydroxide solution was added to the reaction solution, which was then extracted three times with 100 mL of ethyl acetate . The organic phase was dried by magnesium sulfate anhydride, and then the dried organic phase was dried at 40°C or less under reduced pressure to remove a solvent to thereby obtain 20.8 g of
4-[(8-hydroxy-1-octyl)oxy]-3-methylpyridine-1-oxide as an oily substance.

### (2) Production of 4-[(8-acetoxy-1-octyl)oxy]-2-keto-3-methylpyridine

189.4 g (1.855 mol, 27 equivalent) of acetic acid anhydride was added to 20.4 g (69.1 mmol, 1.0 equivalent) of 4-[(8-hydroxy-1-octyl)oxy]-3-methylpyridine-1-oxide to react at 117°C for 7 hr. 175 mL of ethyl acetate and 26 mL of methanol were added to the concentrated residue obtained after acetic acid anhydride was removed by distillation and the obtained mixture was further refluxed with stirring for 5 hr. After the reaction solution was cooled, it was distilled at 40°C or less under reduced pressure to remove a solvent. The obtained concentrated residue was refined by silica gel column chromatography to obtain 1.63 g of
4-[(8-acetoxy-1-octyl)oxy]-2-keto-3-methylpyridine as an oily substance.

### (3) Production of 4-[(8-acetoxy-1-octyl)oxy]-3-methylpyridine-2-thione

1.10 g (2.7 mmol, 1.0 equivalent) of a Lawesson' s reagent and 9.4 mL of toluene were added to 1.60 g (5.4 mmol, 1.0 equivalent) of
4-[(8-acetoxy-1-octyl)oxy]-2-keto-3-methylpyridine and the mixture was stirred under heating/refluxing for 14 hr and 30 min. Because the reaction was not completed, a Lawesson's reagent was added three times in total sequentially while monitoring the progress of the reaction. In the first addition, a Lawesson's reagent was added as follows: amount of the reagent: 1.10 g (2.7 mmol, 1.0 equivalent), reaction under heating/refluxing for 8 hr. In the second addition, a Lawesson's reagent was added as follows: amount of the reagent: 0.44 g (1.1 mmol, 0.4 equivalent), reaction under heating/refluxing for 3 hr and 30 min. In the third addition, a Lawesson's reagent was added as follows: amount of the reagent: 0.44 g (1.1 mmol, 0.4 equivalent), reaction under heating/refluxing for 15 hr. The reaction solution was cooled and distilled at 40°C or less under reduced pressure to remove a solvent and the concentrated residue was refined by silica gel column chromatography to obtain 0.185 g of
4-[(8-acetoxy-1-octyl)oxy]-3-methylpyridine-2-thione as powders.

### (4) Production of 2-[[4-(8-hydroxy-1-octyloxy)-3-methyl-2-pyridyl]thio]-methy 1]-1H-benzimidazole

180 mg (0.58 mmol, 1.0 equivalent) of 4-[(8-acetoxy-1-octyl)oxy]-3-methylpyridine-2-thione and 96.2 mg (0.58 mmol, 1.0 equivalent) of 2-(chloromethyl)benzimidazole were added in a mixed solution of 0. 051 mL of an aqueous 2 mol/L sodium hydroxide solution and 4.4 mL of ethanol, and the mixture was stirred at 50°C for 3 hr. Because the reaction was not completed, 0.289 mL of an aqueous 2 mol/L sodium hydroxide solution was added to the reaction mixture, which was further reacted at 20°C for 14 hr. After the reaction solution was concentrated at 40°C or less under reduced pressure, 7 mL of water was added to the reaction solution, which was then extracted with dichloromethane (30 mL, 12 mL x 2) sequentially. After the organic phase was dried by magnesium sulfate anhydride, the solvent was concentrated to dryness. The obtained residue was refined by silica gel column chromatography. A residue obtained by concentrating the target fraction after the chromatography was crystallized from ethanol, n-hexane and t-butyl methyl ether to obtain 75.5 mg of a colorless crystal of the target
2-[[4-(8-hydroxy-1-octyloxy)-3-methyl-2-pyridyl]thio]-methy 1]-1H-benzimidazole.
The total yield was 0.34% and the purity measured by HPLC was 99.4%.

¹H-NMR (400 MHz, DMSO-d₆) δ:
1.28-1.42 (10H, m), 1.69-1.76 (2H, m), 2.07 (3H, s), 3.31-3.38 (2H, m), 4.06 (2H, t J = 6.4 Hz), 4.66 (2H, s), 6.87 (1H, d J = 6.0 Hz), 7.11-7.15 (2H, m), 7.48 (2H, bs), 8.26 (1H, d J = 5.6 Hz), 12.23 (1H, bs)
MS m/z : 399 (M⁺)

### Example 14

### Production of 2-[[[4-(8-methoxy-1-octyloxy]-3-methyl-2-pyridyl]thio]-methyl]-1H-benzimidazole hydrochloride

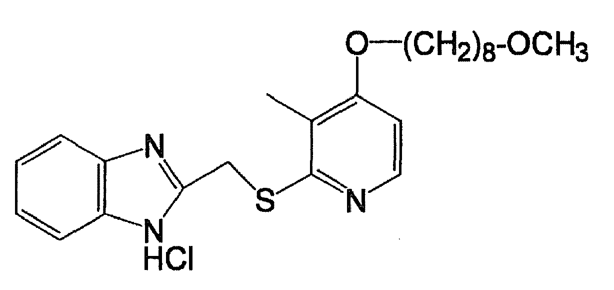

### (1) Production of 4-[(8-methoxy-1-octyl)oxy]-3-methylpyridine-1-oxide

8.6 g (59.9 mmol, 1.0 equivalent) of 4-chloro-3-methylpyridine-1-oxide and 4.8 g (120 mmol, 2.0 equivalent) of sodium hydroxide were added to 33.0 g (205.9 mmol, 3.4 equivalent) of 8-methoxy-1-octanol and 60 mL of toluene and the mixture was refluxed with stirring for 6 hr, and then the reaction was stopped. After the reaction solution was cooled, 100 mL of water and 7.35 g of concentrated hydrochloric acid were added to the reaction solution, and then 0 .14 g of an aqueous 30% sodium hydroxide solution was added. The reaction solution was then extracted three times with ethyl acetate (once with 150 mL thereof and twice with 100 mL thereof). The organic phase was dried by magnesium sulfate anhydride, and then the dried organic phase was dried at 40°C or less under reduced pressure to remove a solvent to thereby obtain 26.02 g of 4-[(8-methoxy-1-octyl)oxy]-3-methylpyridine-1-oxide as an oily substance.

### (2) Production of 4-[(8-methoxy-1-octyl)oxy]-2-keto-3-methylpyridine

161.78 g (1.585 mol, 16 equivalent) of acetic acid anhydride was added to 26.02 g (97.3 mmol, 1.0 equivalent) of 4-[(8-methoxy-1-octyl)oxy]-3-methylpyridine-1-oxide to react at 110°C for 6 hr. 162 mL of ethyl acetate and 30 mL of methanol were added to the concentrated residue obtained after acetic acid anhydride was removed by distillation and the obtained mixture was further refluxed with stirring for 3 hr. After the reaction solution was cooled, it was distilled at 40°C or less under reduced pressure to remove a solvent. The obtained concentrated residue was refined by silica gel column chromatography to obtain 2.18 g of
4-[(8-methoxy-1-octyl)oxy]-2-keto-3-methylpyridine as an oily substance.

### (3) Production of 4-[(8-methoxy-1-octyl)oxy]-3-methylpyridine-2-thione

1.59 g (3.9 mmol, 1.0 equivalent) of a Lawesson's reagent and 14 mL of toluene were added to 2.1 g (7.9 mmol, 1.0 equivalent) of
4-[(8-methoxy-1-octyl)oxy]-2-keto-3-methylpyridine and the mixture was stirred under heating/refluxing for 6 hr. Because the reaction was not completed, a Lawesson' s reagent was added two times in total sequentially while monitoring the progress of the reaction. In the first addition, a Lawesson's reagent was added as follows: amount of the reagent: 0.95 g (2.3 mmol, 0.6 equivalent), reaction under heating/refluxing for 1 hr. In the second addition, a Lawesson' s reagent was added as follows: amount of the reagent: 0.95 g (2.3 mmol, 0.6 equivalent), reaction under heating/refluxing for 3 hr and 30 min. The reaction solution was cooled and distilled at 40°C or less under reduced pressure to remove a solvent and the concentrated residue was refined by silica gel column chromatography to obtain 0.565 g of
4-[(8-methoxy-1-octyl)oxy]-3-methylpyridine-2-thione as powders.

### (4) Production of 2-[[4-(8-methoxy-1-octyloxy)-3-methyl-2-pyridyl]thio]-methy 1]-1H-benzimidazole

565 mg (2.0 mmol, 1.0 equivalent) of 4-[(8-methoxy-1-octyl)oxy]-3-methylpyridine-2-thione and 363 mg (2.2 mmol , 1.1 equivalent) of 2- (chloromethyl) benz imidazole were added in a mixed solution of 1.09 mL of an aqueous 2 mol/L sodium hydroxide solution and 15.2 mL of ethanol, and the mixture was stirred at 50°C for 3 hr. After the reaction solution was concentrated at 40°C or less under reduced pressure, 24 mL of water was added to the reaction solution to extract the solution with 106 mL of dichloromethane and then with 53 mL of dichloromethane sequentially. After the organic phase was dried by magnesium sulfate anhydride, the solvent was concentrated to dryness. The obtained residue was refined by silica gel column chromatography. A residue obtained by concentrating the target fraction after the chromatography was crystallized from ethanolic hydrochloric acid to obtain 168 mg of a crystal of the target
2-[[4-(8-methoxy-1-octyloxy)-3-methyl-2-pyridyl]thio]-methy 1]-1H-benzimidazole hydrochloride.
The total yield was 0.65% and the purity measured by HPLC was 94.7%.

¹H-NMR (400 MHz, DMSO-d₆) δ:
1.29-1.47 (10H, m), 1.71-1.72 (2H, m), 2.11 (3H, s), 3.20 (3H, s), 4.05-4.06 (2H, m), 4.90 (2H, s), 6.85-6.86 (1H, m), 7.52-7.53 (2H, m), 7.74-7.76 (2H, m), 8.13-14 (1H, m)
MS m/z : 413 (M⁺: free body)

### Pharmacological Test 1

### Antibacterial Test

With regard to the compounds produced in Examples 1 to 14, a test for the antibacterial activity of each compound against Helicobacter pylori (H. Pylori) was made.
ATCC43504 which was a standard bacterial strain was used as Helicobacter pylori (H. pylori) to perform the test in vitro in a Columbia agar medium. The bacteria were cultured at 37°C and a pH of 7.0 for three days. The minimum inhibitory concentration (MIC, µg/ml) was found on the fourth day. Each sample was dissolved in 1% DMSO to use. As control drugs for the antibacterial agent, ampicillin (AMPC), clarithromycin (CAM), and gentamycin (GEM) were used. As bacteria for comparison, Gram negative bacteria, for example, E. coli (ATCC 10536 ATCC 25922), Klebsiella pneumonia (ATCC 10031), Proteus vulgaris (ATCC 13315), Pseudomonas aeruginosa (ATCC 9027) and Salmonella typhimurium (ATCC 13311) were used and Gram positive bacteria, for example, Staphylococcus aureus, MRSA (ATCC 33591), Staphylococcus epidermidis (ATCC 12228), Streptococcus pneumonia (ATCC 6301), Mycobacterium ranae (ATCC 110) and Enterococcus faecalis (VRE, ATCC 51575) were used.
The test results are shown in the following Tables 2 and 3.

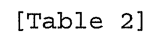

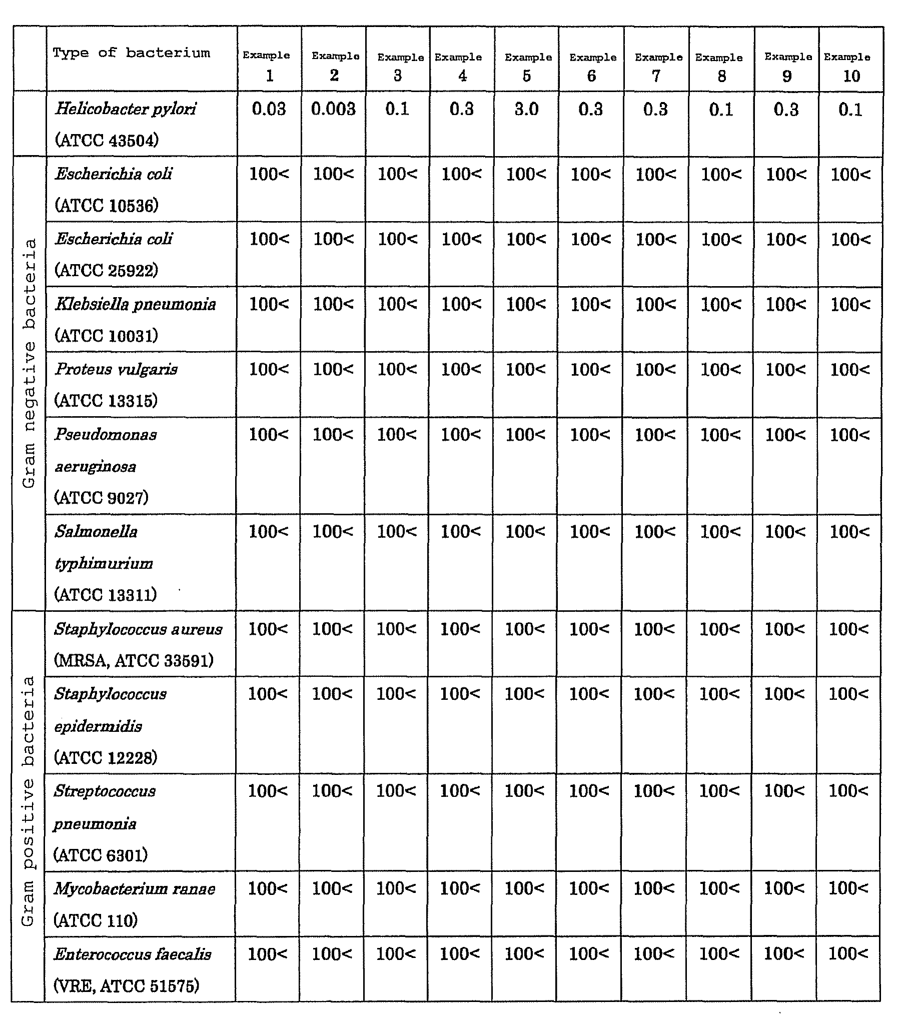

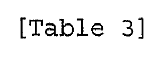

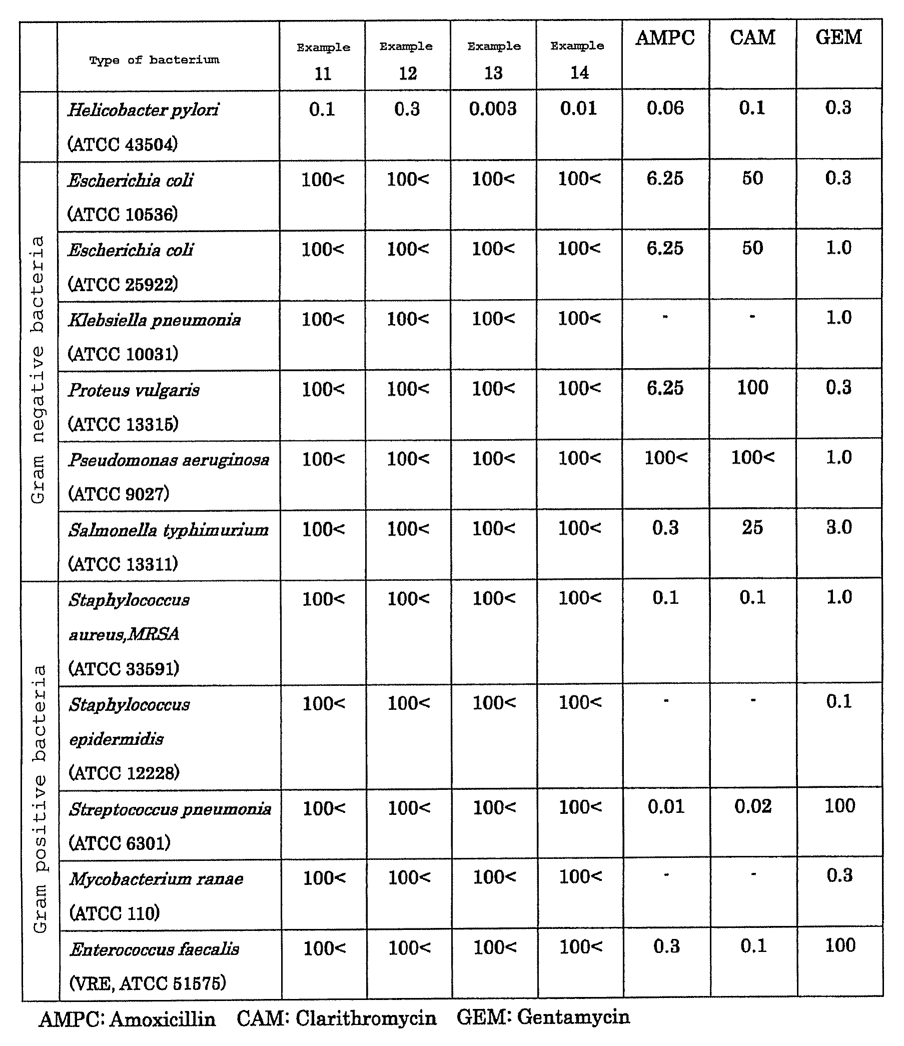

The values in each table show minimum inhibitory concentrations (MIC, µg/ml). "100<" means that any antibacterial ability was not exhibited even at a dose of 100µg/ml.
As is clear from Tables 2 and 3, the novel pyridine thio derivative of the present invention exhibited strong antibacterial ability against Helicobacter pylori (H. pylori) . The MIC of each compound of Examples 1, 2, 13, and 14 was 0.03 µg/ml or less and particularly, the compounds of Examples 2 and 13 had remarkably strong antibacterial ability and the MIC of each compound was 0.003 µg.
All of these compounds of the present invention did not exhibit antibacterial activity with MIC being 100 µg/ml or more against the bacteria such as the Gram negative bacteria or Gram positive bacteria and it was therefore found that the compound of the present invention exhibited selective and specific activity against Helicobacter pylori (H. pylori).
On the other hand, gentamycin (GEM) used as the control drug had a MIC of 0.3 µm/ml against Helicobacter pylori. However, GEM had a MIC of 0.1 to 1. 0 µg/ml against Gram negative or Gram positive bacteria. Further, each result of ampicillin (AMPC) and clarithromycin (CAM) was similar to that of GEM.

### Preparation 1 Tablet

| | |
|---|---|
| Compound of Example 2 | 50.0 mg |
| Mannitol | 65.5 mg |
| Hydroxypropyl cellulose | 2.5 mg |
| Crystal cellulose | 10.0 mg |
| Corn starch | 10.0 mg |
| Carboxymethyl cellulose calcium | 5.0 mg |
| Talc | 2.0 mg |
| Magnesium stearate | 0.2 mg |

The above ingredients were formulated in the above ratios according to the usual method, to prepare a tablet with 145.2 mg/tablet.

### Preparation 2 Granule

| | |
|---|---|
| Compound of Example 4 | 300 mg |
| Lactose | 540 mg |
| Corn starch | 100 mg |
| Hydroxypropyl cellulose | 50 mg |
| Talc | 10 mg |

The above ingredients were formulated in the above ratios according to the usual method, to prepare a granule with 1000 mg/pack.

### INDUSTRIAL APPLICABILITY

The pyridine thio derivative or a pharmacologically acceptable salt thereof according to the present invention exhibits selective antibacterial activity against Helicobacter pylori (H. pylori) without affecting on normal bacteria and is therefore very useful as an antibacterial agent that treats and prevents various diseases in which Helicobacter pylori is involved.

## Claims

1. A pyridine thio derivative represented by the following general formula (I) or a pharmacologically acceptable salt thereof: 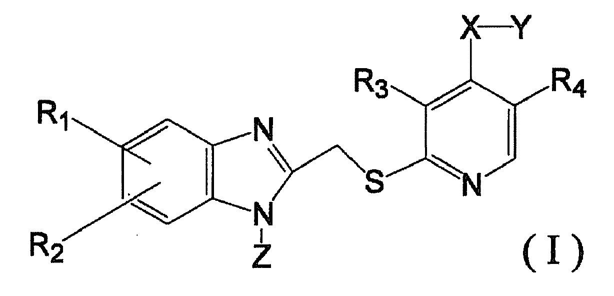 wherein R₁ and R₂ respectively represent a hydrogen atom, a C₁₋₈ alkyl group, a C₁₋₈ alkoxy group, or a halogen atom; R₃ and R₄ respectively represent a hydrogen atom, a C₁₋₈ alkyl group, or a C₁₋₈ alkoxy group; X represents -O-, -S-, or -NH-; Y represents a C₁₋₁₂ alkyl group which may be substituted with a substituent selected from the group consisting of a hydroxyl group, a C₁₋₈ alkoxy group, and a halogen atom, or -(R⁵-O)ₙ-R⁶ (where R⁵ represents a C₁₋₅ alkylene group, R⁶ represents a C₁₋₈ alkyl group which may be substituted with a substituent selected from the group consisting of a hydroxyl group, a C₁₋₈ alkoxy group, and a halogen atom, and n denotes an integer from 1 to 10); and Z represents a hydrogen atom or a C₁₋₈ alkyl group.

2. The pyridine thio derivative or a pharmacologically acceptable salt thereof according to Claim 1, wherein Y in the general formula (I) is a C₃₋₁₀ alkyl group which may be substituted with a substituent selected from the group consisting of a hydroxyl group or a C₁₋₈ alkoxy group.

3. The pyridine thio derivative or a pharmacologically acceptable salt thereof according to Claim 1, wherein Y in the general formula (I) represents-(R⁵-O)ₙ-R⁶(where R⁵ represents a C₁₋₅ alkylene group, R⁶ represents a C₁₋₈ alkyl group which may be substituted with a substituent selected from the group consisting of a hydroxyl group, a C₁₋₈ alkoxy group, and a halogen atom, and n denotes an integer from 1 to 10), in which R⁵ is an ethylene group and R⁶ is a C₁₋₈ alkyl group which may be substituted with a fluorine atom.

4. The pyridine thio derivative or a pharmacologically acceptable salt thereof according to any one of Claims 1 to 3, wherein X in the general formula (I) is -O- or -S-.

5. The pyridine thio derivative or a pharmacologically acceptable salt thereof according to any one of Claims 1 to 4, wherein R₃ and R₄ in the general formula (I) respectively represent a hydrogen atom, a methyl group, or a methoxy group.

6. A pharmaceutical composition, comprising: the pyridine thio derivative or a pharmacologically acceptable salt thereof according to any one of Claims 1 to 5, and a pharmaceutically acceptable carrier.

7. The pharmaceutical composition according to Claim 6, the composition being used to prevent or treat Helicobacter pylori (H. pylori) infections.

8. The pharmaceutical composition according to Claim 6, the composition being used to prevent or treat diseases in which Helicobacter pylori (H. pylori) is involved.

9. The pharmaceutical composition according to Claim 8, wherein the diseases in which Helicobacter pylori (H. pylori) is involved are gastritis, gastric ulcer, duodenal ulcer, non-ulcer dyspepsia syndromes, gastric MALT lymphoma, hyperplastic gastric polyps, gastric cancers, gastrointestinal cancers, pancreatitis, or inflamed intestinal diseases.
